(19) Europäisches Patentamt
European Patent Office
Office européen des brevets

(11) EP 1 920 246 B1

(12) **EUROPEAN PATENT SPECIFICATION**

(45) Date of publication and mention of the grant of the patent:
**10.03.2010 Bulletin 2010/10**

(21) Application number: **05781688.6**

(22) Date of filing: **31.08.2005**

(51) Int Cl.:
***G01N 33/49*** (2006.01) ***B01L 3/00*** (2006.01)

(86) International application number:
**PCT/EP2005/009382**

(87) International publication number:
**WO 2007/025559 (08.03.2007 Gazette 2007/10)**

(54) **COAGULATION TEST SYSTEM**

GERINNUNGSTESTSYSTEM

TEST DE COAGULATION

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**14.05.2008 Bulletin 2008/20**

(73) Proprietor: **Egomedical Technologies AG**
**9242 Oberuzwil (CH)**

(72) Inventors:
- **STIENE, Matthias**
**82205 Gilching (DE)**
- **JONES, Eurig, Wyn**
**81377 München (DE)**
- **NINCIC, Sladjana**
**81377 München (DE)**
- **HORSTKOTTE, Elke**
**81245 München (DE)**

(74) Representative: **Graf von Stosch, Andreas et al**
**Graf von Stosch**
**Patentanwaltsgesellschaft mbH**
**Prinzregentenstrasse 22**
**80538 München (DE)**

(56) References cited:
**WO-A-00/56808**
**WO-A-93/22453**
**WO-A-02/076878**
**US-A- 4 849 340**
**US-A- 5 628 961**
**US-A- 5 629 209**
**US-A1- 2003 210 287**
**US-A1- 2004 028 566**
**US-A1- 2004 031 688**

- **HAIDEKKER M.A., BRADY T.P., CHALIAN S.H., AKERS W.,LICHLYTER D.,THEODORAKIS E.A.: "Hydrophilic molecular rotor derivatives - synthesis and characterization" BIOORGANIC CHEMISTRY, [Online] vol. 4, no. 32, August 2004 (2004-08), pages 274-289, XP002369105 Retrieved from the Internet: URL: www.sciencedirect.com> [retrieved on 2004-05-13]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 920 246 B1

## Description

## Field of the Invention

The invention relates to a coagulation test system for measuring the coagulation of blood in a physiological sample fluid.

## Background of the Invention

The process of blood coagulation is complex and involves a large number of blood components including the generation of fibrin fibres. The fibres are formed by the polymerization of molecules of a protein called fibrinogen. Fibrinogen is catalyzed from an enzyme called thrombin, which is itself catalyzed from the enzyme prothrombin.

The prothrombin time test (PT test) is commonly employed in hospitals, clinics and laboratories to ascertain the ability of a blood sample to clot. The test is extensively used for pre-operative evaluations and for anti-coagulant therapy administered to cardiac patients, for example. The PT test is based upon the length of time required for a sample of blood to clot under the influence of certain reagents such as calcium ions and thromboplastin.

Similarly, individuals suffering from cardiac and vascular diseases and/or having mechanical heart valves are often treated with a daily regimen of blood thinning drugs commonly referred to as anticoagulants. The amount of anticoagulant in the blood stream, to be effective, must be maintained at a level deemed to be proper by a physician. The consequence of improper amounts of anticoagulant in the blood stream is serious, leading to strokes or haemorrhages.

Patients achieving this balance must endure frequent, costly and inconvenient visits to a clinic where the blood's ability to clot can be closely monitored. The monitoring is undertaken with periodic PT measurements as measured by the International Normalized Ratio (INR). For example, an INR greater than 3 results in a higher risk of serious haemorrhage, whilst an INR of 6 increases the risk of developing a serious bleed nearly 7 times that of someone with an INR below 3. In contrast, an INR below 2 is associated with an increased risk of stroke. Therefore, monitoring of the prothrombin time is recommended to ensure that the dose levels are within the therapeutic range.

By monitoring components such as fibrinogen and prothrombin levels within the blood, a physician may acquire meaningful data concerning a patient's blood clotting abilities or other clinical conditions. The proteins that are involved in the clotting (coagulation) process are commonly referred to as factors. The factors are numbered I-XIII, and reference to a factor by its number identifies the corresponding protein to those skilled in the art.

The activation of prothrombin occurs as a result of the action of blood clotting Factor Xa, which is formed by the activation of Factor X during proteolysis. There are two molecular pathways that lead to the activation of Factor X to give Xa, generally referred to as the extrinsic and intrinsic pathways for blood clotting. The extrinsic pathway utilizes only a tissue factor specific to an injured membrane while the intrinsic pathway utilizes only factors internal to the circulating blood. Both of these pathways originate with the interaction of enzymes involved in the blood clotting process with surface proteins and phospholipids.

Various tests have been introduced to measure the coagulation process in both the extrinsic and intrinsic pathways of a patient's blood sample. For example, the Activated Partial Thromboplastin Time (APTT) test measures the coagulation factors of the intrinsic pathway. These factors include Factors XII, XI, X, IX, VIII, V, II and I which may be abnormal due to heredity, illness, or the effects of heparin therapy. Thus, the APTT test is useful as a pre-surgical screen and for monitoring heparin therapy. Similarly, the testing of the fibrinogen polymerization rate using a Thrombin Time (TT) test or a quantitative fibrinogen test providing useful diagnostic data for patients on Warfarin therapy (brand name: Coumadine®) or related pharmaceuticals.

As mentioned previously, the test most commonly used to monitor anticoagulant therapy is the one-stage prothrombin time test. The reaction measured by the PT test is:

$$\text{Blood} + \text{Thromboplastin} + \text{Ca}^{++} \rightarrow \text{Fibrin Clot}$$

Thromboplastin is a phospolipid-protein preparation that activates clotting in blood specimens. Thromboplastins are commercially available from different manufacturers and can be obtained from lung, brain, or placenta extracts and also be synthetically manufactured. Generally, PT values between different laboratories are not in concordance, thus making such values unacceptable for defining therapeutic ranges for anticoagulant therapy.

An International Normalized Ratio (INR) was therefore developed and adopted by the World Heath Organisation in the early 1980's. The object of the normalised ratio was to standardise results from various thromboplastins and coagulation analyzers to become equivalent. Consequently, under the ratio a manufacturer assigns an International

Sensitivity Index (ISI) to each batch of thromboplastin which indicates the relative sensitivity of the thromboplastin compared to an international reference thromboplastin. For example, if a thromboplastin has the same sensitivity as the reference thromboplastin, then the ISI is 1.0. An ISI value greater than 1,0 indicates that a thromboplastin is not as sensitive as the reference thromboplastin. The equation below is used to calculate the INR value using a PT value and a ISI value:

$$INR = \left( \frac{PT_{Patient}}{PT_{mean-normal}} \right)^{ISI}$$

The mean normal PT is determined in each laboratory by averaging the PT values from a number of healthy individuals.

The detection of the formation of fibrin clots date back to the mid 1850's and early methods were manual. By 1910, an apparatus to determine the change in viscosity of a blood sample as it underwent clotting was developed. The apparatus provided a direct indication of voltage which could be plotted against clotting time. In the 1920's, photoelectric techniques became prominent to detect variations in light transmittivity of a blood sample during clotting with variations in the optical transmittivity of the sample observed by a galvanometer. Further investigations of the coagulation of blood plasma using improved photoelectric techniques were conducted in the mid 1930's with optical density increasing as blood coagulated being observed. This led to the development of an instrument which displayed increasing density as a clot formed.

Modern optical density detection systems therefore operate on the principle that an increase in the optical density of a coagulating sample decreases the transmittivity of light through the sample. In a typical optical density detection system, a test blood sample is placed in a transparent sample cuvette and reacted with a coagulation stimulating reagent such as thromboplastin. Light or electro-magnetic radiation in the visible or near-infrared spectrum is then passed through the plasma-reagent mixture as the sample clots. As the biochemical change leading to fibrin formation takes place within the sample, the optical density of the sample increases. Output voltages corresponding to the optical density of the sample enables, after processing with a processing unit, to determine the coagulation of the sample.

While the existence of the relationship between fibrinogen (fibrin) levels and optical density has long been recognized, there has been wide disagreement concerning the nature and proper methodology for measuring the relationship, and numerous test parameters have been devised for determining fibrinogen levels using optical density data.

Further, the increased awareness about the negative effect of irregular blood coagulation time, the acceptance of self-monitoring and self-treatment has led to the development of a multitude of blood coagulation monitors and methods for personal use and point of care testing. However, these devices still lack the development state, economy, and convenience known form home glucose monitoring systems for diabetes patients.

An exemplary method and system for measuring blood coagulation time is disclosed in United States Patent 4,252, 536. The method involves providing a mixture of a blood sample and a reagent, irradiating the mixture with light and detecting the amount of light scattered from the irradiated mixture producing an electrical signal representative thereof. Subsequently, a determination is made from the electrical signal a time at which the most rapid change in electrical signal is occurring and then determining as the end point at a time prior to the first time at which a change which 1/n that of the most rapid change occurred, where n is greater than 1. Most of the methods of measuring coagulation time are based on plasma being introduced into a cuvette and to analyse the properties of coagulation over a period of time.

European Patent Application 1,162,457 discloses a testing system for determining an appropriate coagulation promoting substance for administration to a patient as a therapy for improving clotting function using three sample wells to receive a selected amount of blood.

United States Patent 6,066,504 discloses an electrode assembly which provide quantitative measurement of viscosity changes over intervals of time to signal the coagulation or lysis of a blood sample.

European Patent 974,840 discloses fluidic diagnostic device for measuring an analyte concentration or property of a biological fluid using optical detection means.

PCT WO 20047/044560 discloses a photometric determination of coagulation time in undiluted whole blood having a contained for receiving a sample of undiluted whole blood, a light emission source for emitting light and a light detector for measuring an amount of light from said container.

US Patent 6,084,660 discloses a fluidic medical diagnostic device having at one end a sample port for introducing a sample and at the other end a bladder for drawing the sample to a measurement area, which measures an analyte concentration or a physical property of whole blood, particularly the coagulation time, only after first ensuring that a

whole blood sample has been introduced into the device.

PCT WO2002/086472 discloses the use of fluorescent molecular rotors which vary in fluorescence intensity based on viscosity of the environment. The inventor further relates to a class of molecular motors that at modified with a hydrocarbon chain or hydrophilic group to allow for the measurement of membrane or liquid viscosity.

US Patent Application Publications US 2002/0110486A1 and US 2003/0031594 A1 disclose a test stop comprising a plurality of reaction zones utilised for quality assurance purposes. The test strip requires a volume of about 20μL blood. However, if a user has to test frequently, as required for proper management of coagulation therapy, these large sample volumes are unpractical and disadvantageous.

PCT/EP 2004002284 discloses a dry reagent test element for the photometric detection and quantitative determination of an analyte, e. g. glucose, in a physiological fluid, e. g. blood, having a sample distribution system with at least two detection areas which is provided with an integrated calibration system and which requires very small sample volumes of about 0,5 μL.

United States Patent Application Publication US 2003/210287 discloses a medical diagnostic device adapted for measuring blood coagulation times that has a non-absorbent substrate upon which a reagent is deposited by non-impact printing of microdroplets. The device comprises a bladder to create a suction force to draw a sample into the device, and a stop junction to stop flow after filling the reagent area. The top layer preferably has a hydrophobic surface facing the substrate, at least in the channel and reagent areas.

International Patent Application Publication WO 00/56808 discloses a method for rendering a surface covered by a polymer material more hydrophilic by treatment in a gas plasma of a non-polymerizable gas. Moreover, WO 00/56808 describes a liquid transportation system created by a surface (I) exhibiting a hydrophilic pattern and a surface (II) exhibiting hydrophobic character or having a hydrophilic pattern matching the hydrophilic pattern of surface (I). In addition, WO 00/56808 provides a kit containing a device comprising a liquid transportation system, and a fluorescent substance to be detected in the device.

International Patent Application Publication WO 02/076878 describes a method and structure for microfluidic low guiding, which employs wettable and non-wettable surfaces to form a flow-path that has low flow resistance, promote laminar flow and provides a superior gas-liquid interface.

United States Patent Application Publication US 2004/031688 provides microchip laboratory systems, such as actuators, for manipulating small quantities of liquids (microfluidics) based on the phenomenon of electrowetting. The microchip laboratory system comprises a material handling device that transports liquid in form of individual droplets positioned between two surfaces. The formation and movement of droplets are controlled by plurality of electric fields across the gap between the two surfaces, the electric fields effecting a hydrophobic-hydrophilic conversion of the droplets.

However, up to now no test system exists, which is suitable for measurement of coagulation of a blood sample and which is provided with integrated quality control means and requires only small sample volumes.

Therefore, it is the object of the present invention to provide a test system for determining the coagulation of whole blood which requires only minimal steps, such as the application of blood onto a strip, which provides a subsequent antomatic calculation of an accurate test result including a means for 'on-strip' quality control and which requires only a small sample amount.

It is a further object of the present invention, to provide a production process for a coagulation test element which does not involve many and complicated production steps and therefore is inexpensive and usable for products assisting patients in self-monitoring blood coagulation and/or in a physician's place of work.

## Summary of the invention

Thus, the present invention provides a test element for the determination of coagulation in a plasma or whole blood sample having a first surface and a second surface in a predetermined distance opposite from each other, said both surfaces being provided with two substantially equivalent patterns forming areas of high and low surface energy which are aligned mostly congruent, whereby the areas of high surface energy create a sample distribution system with at least two detection areas, wherein at least one of the detection areas of the first and/or second surfaces is provided with at least one coagulation stimulation reagent.

In another aspect the present invention provides a method for preparing a coagulation test element.

In a further aspect the present invention provides a coagulation test system consisting of a coagulation test element and a meter device for performing blood coagulation assays using a simplified format to provide a verified result in accordance with worldwide standards by providing on strip quality control.

A better understanding of the features and advantages of the present invention will be obtained by reference to the following detailed description of illustrative and preferred embodiments in conjunction with the attached drawings.

## Brief description of the drawings

Figure 1 shows a perspective view of one embodiment of the coagulation test element of the present invention provided in shape of a test strip.

Figure 2 shows a perspective view of the embodiment according to Figure 1, showing the sample distribution enlarged.

Figure 3 shows an exploded perspective view of the device according to Figure 1, showing the three layers separately.

Figure 4 shows different forms of the discontinuity of the centre layer forming the sample cavity together with the first and second surface.

Figure 5a is a sectional view of a detection area of the sample distribution system constructed by hydrophobic guiding elements.

Figure 5b is a sectional view of another embodiment of a detection area of the sample distribution system using hydrophilic pathways.

Figure 6 shows different embodiments of the sample distribution system with different patterns of pathways and detection areas suitable for different evaluation methods.

Figure 7a shows the sample distribution system of Figure 5b in conjunction with a light emitter and detector arrangement in a section view suitable to evaluate the changes in light absorbance of the sample.

Figure 7b shows the sample distribution system of Figure 5b in conjunction with a detector means configured to evaluate the changes in the fluorescence signal of a molecular rotor added to the sample or to evaluate the turbidity of the supplied sample fluid.

Figure 8 shows different molecular rotors and their molecular structure;

Figure 9 is a graph showing the schematic evaluation of coagulation results with implemented positive and negative quality controls.

Figure 10 shows the optical spectrum of whole blood from 500 to 700 nm.

Figure 11 provides a graph displaying the progress of a blood coagulation reaction initiated with Thromborel S® and monitored at 600 nm.

Figure 12 shows a simplified block diagram of an example meter for use in a method of the invention.

Figure 13 shows the influence of registration failures during the lamination process on the sample volume of the test element and the top respectively the sectional view of an alternative embodiment, which allows higher tolerances for the registration of base and cover layer without compromising on the test strip quality.

Figure 14 shows the production steps of the coagulation test elements with strip shape.

## Detailed description of the invention

As shown in Figure 1 and Figure 2, the coagulation test strip 1 of the present invention is a multiple layer arrangement comprising a base layer 2, a centre layer 3 overlaying the base layer 2, and a cover layer 4 overlaying the centre layer 3. The centre layer 3 presents a discontinuity 5, which creates a hollow cavity in conjunction with the base layer 2 and the cover layer 4. Within said cavity there is located a sample distribution system 6 which is connected to a sample application area 9 located on one side of the coagulation test strip. The sample application area 9 as interface to the user is preferably formed by a convex curve 10 extending from one major side of the coagulation test strip for easier application of the sample. Opposite to the sample application area 9, 10 on the second major side of the coagulation test strip is the location of an air vent 11 allowing the displacement of air while the physiological or aqueous fluid is distributed to the predetermined detection areas 6a, 6'a (see Figure 3). It might be noted that the construction requires only one air vent independent of the amount of predetermined detection areas used within the coagulation test element. The described elements of the sample distribution system with areas of high surface energy, sample application area, air vent, centre layer and discontinuity in the centre layer form the totality of the coagulation test element, which creates the intrinsic capillary action to exert the distribution of the applied physiological or aqueous fluid to the predetermined detection areas.

In addition, the coagulation test strip 1 possesses registration features 7, 8 useful to differentiate between several kinds of test strips for the determination of different parameters such as Prothrombin Time (PT) and Activated Partial Thromboplastin Time (APTT). By this means, a multi analyte meter could be instructed to run a special program or procedures with selectable parameters upon strip insertion required for the determination of different parameters. As illustrated in Figure 3, which represents the multi-layer arrangement of Figure 1 and 2 in an exploded view, the base layer 2 provides a first surface 2a, and the cover layer 4 provides a second surface 4a. The first surface 2a and the second surface 4a are patterned with areas which will create the sample distribution system 6. The pattern of the sample distribution system 6 comprises a predetermine number of detection areas 6a and sample pathways 6b, which are aligned and registered mostly congruent upon assembly of the multi-layer arrangement. The centre layer 3 defines the

distance between the first surface 2a of the base layer 2 and the second surface 4a of the cover layer 4 and has a discontinuity 5 to form a hollow cavity together with the first surface 2a of the base layer 2 and the second surface 4a of the cover layer 4. The sample distribution system 6 which will be formed between the first surface 2a and second surface 4a is located within the cavity created by the discontinuity 5 of the centre layer 3 and the first surface 2a of the base layer 2 and the second surface 4a of the cover layer 4. Preferably, the hollow cavity is substantially larger by design than the sample distribution system.

Since the purpose of the discontinuity 5 of the centre layer is only to create a cavity for the sample distribution system 6, the discontinuity 5 of the centre layer 3 can have different forms; examples thereof are shown in Figure 4. Figure 4a shows an umbrella shaped coagulation test element cavity 12. Figure 4b shows a rectangular coagulation test element cavity 13, and in Figure 4c the sample cavity 14 has a circular shape. The discontinuity 5 of the centre layer 3 does not influence the size of the predetermined detection areas 6a and the size of the pathways 6b of the sample distribution system 6 and therefore does not influence or change the required sample volume. Compared to the sample distribution system 6, the cavity shapes shown in Figure 4 are rather simple, thus allowing the application of simple punch tools and fast processing with less demand on the registration accuracy.

The sample distribution system 6 located in the cavity formed by the discontinuity 5 of the centre layer 3 and the first surface 2a of the base layer 2 and the second surface 4a of the cover layer 4 is formed by creating areas of high and low surface energy on said surfaces 2a and 4a. The areas of high and low surface energy on the first surface 2a of the base layer 2 and the second surface 4a of the cover layer 4 are aligned and registered mostly congruent to each other. Since the applied physiological fluid or any other aqueous sample is wetting only the areas with high surface energy, it is thus constrained within the predetermined flow paths 6b and detection areas 6a of the sample distribution system 6 and between the first surface 2a of the base layer 2 and the second surface 4a of the cover layer 4.

Figure 5a shows a construction of the sample distribution system 6 using hydrophobic "guiding elements". In this embodiment of the coagulation test element of the present invention the base layer 2 and the cover layer 4 are coated with a hydrophobic layer 16, except the areas, which will form the sample pathways and detection areas. The hydrophobic layer 16 creates an area with low surface energy, which will exert a repellent force onto an applied sample fluid 15 and constrain the sample fluid 15 therefore to the areas of high surface energy which will form the sample distribution system 6.

Preferably, the hydrophobic layer is applied on a hydrophilic surface, which is wettable by the physiological or aqueous fluid. The procedure described above requires a hydrophilic surface, which can be produced from a natural hydrophilic polymer such as cellophane or glass as well as from a hydrophobic surfaces of common polymers (examples are given below) by rendering the hydrophobic surface hydrophilic using a coating process or physical or chemical plasma deposition of hydrophilic monomers that can be vaporised in vacuum, e. g. silicon dioxide, ethylene oxide, ethylene glycol, pyrrole or acrylic acid. Subsequently, the pattern of "guiding elements" can be realized by printing hydrophobic ink on the hydrophilic surfaces of the base and cover layers.

A suitable hydrophobic ink will have contact angles with water of typically more than 100° and a surface energy of typically less than 25 mN/m and contain typically monomers, oligomers, and polymers with hydrophobic functions, hydrophobing additives, or hydrophobic pigments and fillers.

Figure 5b shows another construction of the sample distribution system using hydrophilic pathways. In this embodiment of the coagulation test element the base layer 2 and the cover layer 4 are coated with a hydrophilic layer 17 thereby creating areas of high surface energy.

The hydrophilic layer 17 printed on the hydrophobic surface is highly wettable by a physiological or aqueous fluid; thus, the areas of high surface energy creating the hydrophilic pathways of the sample distribution system will exert a positive capillary force onto the applied physiological or aqueous sample fluid to transport the sample fluid to the separate detection areas.

The hydrophilic layer 17 can be realized by printing hydrophilic or amphiphilic agents on a hydrophobic surface. Inks with hydrophilic functions can be realised from a wide selection of high molecular weight water and alcohol soluble polymers and mixtures thereof. Particularly useful are derivatives prepared form alginates, cellulose, hydroxyethyl cellulose, gums, polyalcohols, polyethylene-glycols, polyethylene-oxides, vinylpyrolidone, polystyrene sulfonates, polysulfonates, alkyl-phosphocholine derivates and others; particularly useful are also organo-modified silicone acrylates, which are a cross-linkable species of organo-modified polysiloxanes and fluorinated surfactants. Suitable coatings provide a contact angle with water of typically less than 35° and a surface energy of typically more than 50 mN/m.

The base layer and cover layer suitable as substrates for the printing process may be formed of a material like glass, polyvinyl acetate, poly-methyl-methacrylate, poly-dimethyl-siloxane, polyesters and polyester resins containing fluorene rings, polystyrenes, polycarbonates and polycarbonate-polystyrene graft copolymers, terminal modified polycarbonates, polyolefins, cycloolefins and cycloolefin copolymers, and/or olefin-maleimide copolymers.

In case the substrate has an intermediate hydrophobic character, the printing of hydrophilic pathways with a surrounding hydrophobic pattern, i.e., a combination of the constructions of Figure 5a and Figure 5b, is possible as well.

Moreover, it is possible to physically elevate the areas of high surface energy of first and second surfaces from

the areas of low surface energy by etching, embossing, or simply by printing the hydrophilic layer with about three to five times increased thickness on the first and the second surface. Due to this elevation the capillary gap of the hydrophilic pathways gets smaller in relation to the surrounding area and exerts a higher capillary forth on the sample liquid.

The volume requirement for the sample distribution system contained in the coagulation test element of the preferred embodiment is with about 0.5μL-1.0μL very low and requires only about 100nL - 150nL pet detection area, whether the areas of high and low surface energy are created by hydrophobic guiding elements or hydrophilic pathways or by a combination of both. However, it will be obvious for the one skilled in the art that the volume of the sample distribution system will vary with various designs and with the number of employed predetermined detection areas.

Figure 6 shows different patterns of the sample distribution system, which can be realized by hydrophilic pathways as illustrated in Figure 5b, or by the hydrophobic "guiding elements" as illustrated in Figure 5a, or by a combination of hydrophilic pathways and hydrophobic guiding elements. The selected sample distribution system needs to be appropriate for the selected physiological parameter to be evaluated and for the employed detection chemistry.

Thus, the repetition of sample and standard measurements is possible for particular serum or whole blood samples with the embodiments shown in row II to IV. Likewise, it is possible to use the coagulation test element provided in row IV for the evaluation of two coagulation parameters such as Prothrombin Time and Activated Partial Thrombin Time.

As stated above, the formation of a fibrin clot is dependent on a reaction between Thromboplastin and Calcium ions reacting with blood as shown below:

$$\text{Thromboplastin} + Ca^{++} + \text{Blood (or Plasma)} \rightarrow \text{Fibrin clot} \qquad \text{(Reaction 1)}$$

For Reaction (1) to take place, the detection areas 6'a of the sample distribution system 6 of the first surface 2a of the base layer 2 or the second surface 4a of the cover layer 4 are **characterised in that** they are coated with formulations 18, 19, as shown in Fig. 5a and 5b, which allow the promotion and detection of a coagulation reaction in a blood sample.

In one embodiment of the inventive test element, the formulation 18 contains a coagulation stimulating reagent, such as thromboplastin (e.g. available from Dade Behring Holding GmbH, Höchster Strasse 70, 65835 Liederbach, Germany), whereas formulation 19 contains calcium ions. The coagulation stimulating reagent is a promoter for the coagulation of blood in a detection area thus allowing the detection of the optical properties by transmission or absorbance photometry or light scattering.

The Prothrombin Time or the Activated Partial Thrombin Time can be monitored by change of light absorbance or light scattering. During the coagulation process the Fibrinogen is converted to Fibrin that forces the previously arbitrary distribution of the red blood cells and platelets into a mostly associated stage, whereby the red blood cells and platelets becoming trapped and connected with the Fibrin fibres and each other while forming the blood clot These changes in the physical consistency of the blood sample leads to a reduction of scatter centres and therefore to a change in the light absorbance and turbidity of the examined blood sample. For the evaluation of the changes in light absorbance the detector arrangement shown in Figure 7a is suitable

Figure 7a shows a detector arrangement for measuring the optical density of the sample within the coagulation test element according to Figure 5b. The arrangement includes a light source 20, which emits light 24 of a certain wavelength in direction of the sample detection area. The light emitted from the light source 20 passes through an optical arrangement 21, e.g. a diffuser or lens, and an aperture 22, the base layer 2, the sample 15, and the cover layer 4 of the detection area and is detected on the opposite side of the device by a detector means 23.

The coagulation test element is designed to perform more than one determination to provide additional quality control measurements. In this case, the coagulation test element provides at least two, preferably three coagulation detection areas. Preferably, all of the detection areas 6'a on the first surface 2a are coated with the coagulation stimulating reagent 18 (e. g. Thrombin) promoting the reaction between the chemical components to generate a fibrin clot, whereas one sample detection area, e. g. 6a2, of the second surface 4a is coated with a chemical formulation containing a coagulation accelerator promoting a fast and complete coagulation (positive control), and an other detection area, e. g. 6a3, of the second surface 4a is provided with a chemical formulation containing a coagulation inhibitor suppressing the coagulation of blood (negative control).

For Reaction (1) to take place, the quantities of thromboplastin, calcium ions and, if necessary, quality control formulations, such as a coagulation inhibitor or accelerator, are precisely dosed on said sample detection areas. Preferably, the dosing is performed by drop on demand deposition methods, although other techniques such as ink jet printing would be known to persons skilled in the art. The exact dosing of the coagulation stimulating reagent applied to the sample detection areas is critical for a proper reaction procedure and thus for a reliable calculation of the end point of a coagulation reaction. For instance, in an example embodiment, the amount of thromboplastin can be constant throughout each sample detection area, whilst the concentration of coagulation inhibitor, such as EDTA, can be varied.

In a further embodiment of the inventive test element, in addition to the dosing of thromboplastin, calcium ions and quality control formulations, such as a coagulation inhibitor and accelerator, a further component, which functions as a fluorescence detection aid, can be applied to the sample detection areas of the first and/or second surface(s) 2a, 4a. If said detection areas are supplied with so called fluorescent molecular rotors, the coagulation reaction can be monitored by fluorescence.

Fluorescence is the emission of light from any substance and occurs from the first excited state of a molecule. In the initializing process such a molecule is excited by absorption of light. In the course of the following few nanoseconds the molecule returns to its ground state and gets rid of its excitation energy either by emission of light - called fluorescence - or by movements and rotations of its molecular backbone.

Fluorescence typically occurs from aromatic molecules. Aromatic molecules absorbing visible light in the range between 400 and 800 nm appear coloured. Furthermore, a chromophore is the part of a dye that determines the absorption and emission properties of the whole molecule. The amount or intensity of emission of a specific chromophore is quantified by its fluorescence quantum yield. The fluorescence quantum yield is defined as the number of emitted photons relative to the number of absorbed photons. A wide range of commonly used fluorescence dyes have a fixed quantum yield, whereby all dyes with large quantum yields approaching 100% emission efficiency displays the brightest emissions, such as Sulforhodamine 101 also known as Texas Red.

Dyes carrying flexible groups at the end of their chromophore are known as molecular rotors and show a dependence of their fluorescence quantum yield on the viscosity of the solvent. As the viscosity of the solvent increases, the fluorescence quantum yield of those dyes increases. This effect can be attributed to the mobility of the flexible, non-rigid groups at the end of the chromophore which is lowered by increasing viscosity. The more mobility of the side groups attached to the chromophore is hindered, the more the dye molecule cannot relax to its ground state via movements of its molecular scaffold and gets rid of its excitation energy by emission of light. Examples for fluorescent dyes sensitive to the viscosity of the solvent can be found in the classes of xanthene, oxazine and carbopyronine dyes.

The effect can be attributed to the mobility of the diethylamino groups which is lowered by increasing viscosity. One example of such a dye in the xanthene class is Rhodamine B which is shown as a chemical structure below:

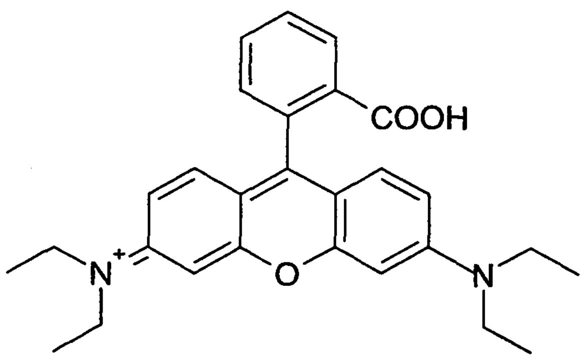

The chemical structure below shows as way of example the mobility of the diethylamino groups which is subsequently lowered by contact to the reagents of reaction 1. Since the reagents lead to the formation of a fibrin clot, i.e. the coagulation of a physiological fluid, the viscosity of the sample increases and subsequently the fluorescence of the molecules. The marked diethylamino-groups at the end of the chromophore are non-rigid and rotate as marked around the bond. As this movement is strongly hindered by increased viscosity because of coagulation, the fluorescence emission of the dye is increased.

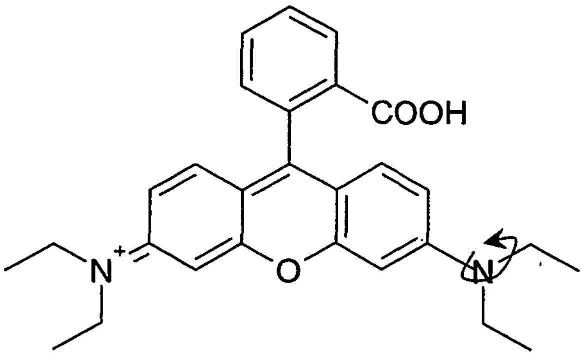

In respect to the disclosed invention fluorescence probes sensitive to change in the viscosity are most useful. Further examples of molecular rotors are Auramine O., Crystal violet 4, p-N,N-dimethylaminobenzonitrile 5, p-N,N-dimethylaminobenzonitrile 6, Julolidinebenzylidenemalononitrile, Rhodamine 19, Rhodamine G6, Rhodamine B, Oxazine 1, Oxazine 4, Oxazine 170. Their molecular structures are shown in Figure 8.

In case the reaction is monitored by fluorescence it is most useful to arrange the light source and the detection means not opposite each other and rather in an angle of approximately 90 degrees to achieve maximum sensitivity, as shown in Figure 7b. The preferred angle between the light source and the detection means is between 80 and 120 degrees but most preferably it is approximately 109 degrees and the coagulation test system is placed in the optical detection arrangement in way that the angle between the base layer and the light source and the angle between the cover layer and detection means is approximately 54 degrees to avoid background noise due to internal reflections on the different surfaces of the detection area (or more generally of the coagulation test system). For full operation said detector 23 is configured in addition to the optical arrangement 21 and 22 with the optical filters 21a and 22a to discriminate between the excitation and the emission wave length, thus the detection means will only see the light 24 emanating from the fluorescence dyes and not the light 24a originated by the light source. Albeit, one skilled in the art will notice that the actual angle has to be optimised for a specific application, the required sensitivity, and the requirements of the meter respectively the detection device.

The coagulation test element 1 has at least one detection area 6a which is required for an accurate prothrombin time measurement, but in a preferred embodiment three detection areas 6a1-6a3 can be utilised. The physical make up of coagulation test element 1 allows flexibility in the composition of compounds applied in various detection areas. For instance, detection areas 6a-6c can have different concentrations of the coagulation stimulating reagent, such as thromboplastin, applied on a first surface 2a of a base layer 2 whilst calcium ions and the fluorescent molecular rotor can be applied to a second surface 4a of a cover layer 4. Alternatively, all reagents can be applied either on the first surface 2a of the base layer 2 or the second surface 4a of the cover layer 4 of the coagulation test element 1.

After the physiological fluid, such as blood or plasma, is applied to the sample application area 9 and distributed to the detection areas by capillary action, it dissolves the coagulation stimulating reagent contained in the formulation 18 on the detection areas of the first surface 2a as well as the molecular rotor and/or a potential coagulation inhibitor such as EDTA contained in the formulation 19 on the predetermined detection areas of the second surface 4a forming a mixture of blood or plasma and coagulation stimulating reagent such as thromboplastin, and calcium ions plus the additional materials provided on the second surface.

Preferably, the coagulation stimulating reagents applied to the predetermined detection areas are readily soluble by a physiological fluid such as blood and positioned close to each other to allow rapid diffusive mixing of all components, thus enabling a fast reaction of the components contained in the detection areas to expedite a fast photometric determination of the forming coagulation reaction.

If there are more than two, preferably three, sample detection areas arranged within the sample distribution system, one, e.g. 6a1, can be used to detect the Prothrombin Time or the Activated Partial Thromboplastin Time. An additionally sample detection area, e.g. 6a2, can be configured to provide a negative control using a coagulation inhibitor on the second surface 4a or omitting the coagulation stimulating agent on the first surface 2a, whereby a further sample detection area, e.g. 6a3, can be configured to provide a positive control using a coagulation accelerator, e.g. a gelling agent mimicking the coagulation of blood even if blood would posses a coagulation deficiency. Thus, the processing means of the measurement device can compare the measurement result of the sample with the two provided standards allowing a clear decision or the indication of an erroneous measurement.

Figure 9 shows a schematic evaluation and measurement of the coagulation time using a molecular rotor as fluorescence probe and detection aid. The figure also shows the comparison of the measurement signal related to a blood sample 27 with a positive standard 26 providing the maximum fluorescence achievable after the full formation of

the blood clot and the comparison with a negative standard 25 providing minimum fluorescence signal related to a non coagulated blood sample. After the application of the whole blood sample onto the sample application area 9 the blood sample 15 is transported by capillary action created by the sample distribution system to the different sample detection areas 6a/6’a shown in Figure 3. The sample will dissolve the coagulation stimulating reagent provided on the first surface 2a of the base layer 2 allowing the coagulation reaction to start immediately after the sample detection area 6’a1 is filled. The detection unit of the measurement device will register the introduction of the blood sample thus the processing means of the detection device can initiate to allow a time resolved evaluation of the coagulation reaction.

As mentioned above, one sample detection area, e. g. 6a2, can be configured as negative standard providing the means of comparing the measurement signal of the blood sample in sample detection area 6a1 with the measurement signal of a blood sample showing no coagulation reaction. Such behaviour can be achieved either by the deposition of non coagulation stimulating reagent in sample detection area 6’a2 or by the deposition of an coagulation inhibitor on sample detection area 6a2. Typical coagulation inhibitors are lithium heparin and sodium respectively the potassium salt of ethylenediaminetetraacetic acid (EDTA).

On the other hand, a positive standard can be realised by accelerating the coagulation reaction or by mimicking the viscosity of a fully formed blood clot with a different cross-linking agent, which provides a faster reaction time than a non pathogen blood sample, thus the positive reference value is achievable before the blood sample in detection area 6a1 is coagulated. This kind of cross-linking can be achieved providing the right concentration of alginate on the second surface 4a of the second layer 4. The alginate will mix with the blood and begin to gel respectively coagulated due to the reaction with the calcium ions inside the blood sample and/or additional calcium ions provided on the first surface of the base layer. However, one skilled in the art will recognize that other gelling agents might be applicable and useful for this reaction as well.

During the reactions the processing means of the measurement device can compare the reading of sample detection area 6a1 with the negative standard 25 and the positive standard 26. As soon as measurement signal of the blood sample, provided by sample detection area 6a1, reaches the same magnitude as the positive standard (indicated with numeral 28 of Figure 9) a processing unit of the measurement device can stop the timer and evaluate the final result. Further the processing unit can perform some additional quality checks to verify that the analysis was performed correctly and provides meaningful data to the user/patient. In this respect, the processing unit can compare the actual measurement values of the positive and negative standards, which needs to be separated by a minimum and pre-programmed value. If the amplitude of both signals becoming to small the device can issue an error message that the determination was not successful. Further the device can calculate the slope 27 of the coagulation reaction and compare it again with some pre-programmed physiological values which describe the most extreme values observed by clinicians.

While monitoring the turbidity of the sample over a wide range of the spectrum, e. g. by using a halogen lamp as light source, it is useful to restrict the monitoring window to a narrow part of the spectrum if the sample changes are monitored by light absorbance. Figure 10 shows a spectrum of whole blood between 500 and 700 nm. The prevalent feature of the spectrum is the haemoglobin double peak 40 between 520 and 600 nm. Principally, one can evaluate the progress of the coagulation reaction by light absorption anywhere in the provided region of the whole blood spectrum it is less demanding on the technical measurement device if the reaction is monitored at a wave length outside of the haemoglobin absorbance range i.e. 600 nm as indicated by numeral 41.

Figure 11 provides an exemplary evaluation of coagulation reaction by light absorption at 600 nm. Blood is introduced in the coagulation test element via the sample application port 9 and the transmission of light is rapidly reduced respectively the absorbance of light is rapidly increased as indicated by numeral 42. Subsequently the coagulation formulation provided in the sample detection areas 6’a of the first surface 2a of the base layer 2 is dissolved and the coagulation reaction is initiated by the reaction of the tissue factor (tissue thromboplastin) with the blood or plasma sample. This point in time is defined as t=0 and the processing unit will start the recording of measurement data. The period of time between the events 42 and 43 can be understood as the lag phase of the reaction, here the full dissolution of the reagents and mixing with the blood or plasma sample is achieved and the tissue thromboplastin triggers a series of coagulation factors of the extrinsic pathway. Showing in the sequence the activation of factor VII, factor X, factor V, factor II. The last stages of the coagulation cascade can be monitored between event 43 and 44 where the fibrinogen is transformed into fibrin. Often the fibrin clot is not stable and starts deteriorating after the plateau 44 is reached. The deterioration rate and quantity depend on the amount of fibrin fibres in clot and varies form patient to patient. Normally, highly viscose blood samples show a slower deterioration then low viscose blood samples.

The result of the measurement generally and the result of the Prothrombin Time according to the above example have to be evaluated between the events 42 - 43 indicating the time period $t_1$ and between the events 43 - 44 indicating the time period $t_2$ following the generally equation 1:

$$PT = f_{PT}(a \cdot [f(t_1)] + b \cdot [f(t_2)])$$ Equation 1

The factors *a* and *b* are required to give a proportional weight to the time periods $t_1$ and $t_2$, which always contribute to different proportions to the result PT given by $f_{PT}$. Whereby $t_1$ is influenced more by the type of the inert ingredients of the coagulation formulation that govern the dissolution of the tissue factor, $t_2$ is mostly influenced by the activity of the applied tissue factor itself and the calcium ion concentration. Additionally, both time period $t_1$ and $t_2$ are modulated by the reaction temperature, which should be ideally set to or close to 37°C, lower temperature regimes will prolong the coagulation time. However, for hand held devices one will always have to find the best solution between portability, energy consumption and laboratory performance.

Figure 12 shows a simplified block diagram of a meter 80 for use in conjunction with the present invention. The meter 80 can be designed around a processing unit such as the MAXQ2000 microcontroller (available from Dallas Semiconductor Corporation, 4401 South Beltwood Parkway, Dallas, Texas, USA). The processing unit 81 can serve the following control functions: (1) timing for the entire system; (2) processing the data from the light detection means; (3) calculating PT time from the measured data; and (4) outputting PT time or INR value to a display means 83. A memory circuit can store data and the processing unit operating program. The display means 83 can take various forms such as liquid crystal or LED display. The meter 80 can also include a start-stop switch and can provide an audible or visible time output to indicate for applying samples, taking readings etc., if desired.

Processing unit 81 may be programmed with software to allow it to make, in conjunction with meter 80 a coagulation measurement. The light emitted from the light source 20 passes through an optical arrangement 21, and detected by a detection means 23. The software programmed into processing unit 81 can further contain an algorithm to calculate the coagulation time as an International Normalised Ratio, formulated in the mid-1980's, to standardise PT values so that results from different thromboplastins and coagulation analysers become equivalent. The expression is given below as:

$$INR = \left( \frac{PT_{Patient}}{PT_{mean-normal}} \right)^{ISI}$$ Equation 2

where ISI is the International Sensitivity Index, PT patient is the time for coagulation for a blood sample from a patient, mean normal PT is the average PT time for around 20 individuals. The ISI value is given by the different manufactures of Thromboplastin.

The method of using coagulation test element 1 of the present invention can be understood with reference to the block diagram of a meter shown in Figure 12. The user inserts a coagulation test element 1 into a strip holder 82 of a meter 80 which is automatically activated by triggering a 'push to make' switch which may be integrated thereon. Registration features designed on element 1 engage with registration features on strip holder 82 to ensure that element 1 is placed in a correct position. Such correct placement of element is of paramount importance to ensure the operation of combined meter 80 and strip 1. Optionally, the meter 80 can be activated by a user pressing a switch. Accordingly, a user performs a finger prick and applies whole blood to application area 9 of inserted coagulation test element 1.

The volume of blood required for a test to take place in the present invention is in the order of 1μL. Since the hydrophilic agent printed on the hydrophobic surface is highly wettable by a physiological or aqueous fluid, the areas of high surface energy creating the hydrophilic pathways of the sample distribution system will exert a positive capillary force onto the applied physiological sample fluid to transport the sample fluid to the separate detection areas. Therefore the physiological sample will rapidly distribute to each sample detection area (6a-c) and activate the coagulation stimulating reagents therein.

Next, coagulation test time starts since the reagent in detection areas 6a-6c aids in the coagulation process allowing the coagulation to take place and the optical properties are processed to give the point at which coagulation has occurred.

**Preparation method of the coagulation test element**

The coagulation test element of the present invention, which is preferably produced in strip form, can easily be prepared by processes to those of ordinary skill in the arts of printing, punching, and laminating. The design of the coagulation test element allows a simple and cost efficient production process, which is preferably but not necessarily of a continuous nature.

In a first step of the preparation method, a pattern of the sample distribution system 6 is formed by creating areas of high and low surface energy on a substrate. In a first embodiment, the areas of high surface energy forming the sample pathways 6b and detection areas 6a, 6'a on the first and second surfaces 2a, 4a are created by applying a hydrophilic formulation on a hydrophobic surface of a substrate. As detailed above, it is also possible to create the areas of high and low surface energy by applying a pattern of hydrophobic "guiding elements" on a hydrophilic surface. In the preferred case the substrate has an intermediate hydrophobic character of commercially available transparent polymer films, whereby areas of low and high surface energy of the sample distribution system and sample detection areas are created by printing the hydrophilic pathways underneath or surrounded by the hydrophobic pattern of the hydrophobic guiding elements.

The substrate may be formed of a material like glass, polyvinyl acetate, poly-methyl-methacrylate, poly-dimethyl-siloxane, polystyrenes, polyesters and polyester resins containing fluorene rings, polycarbonates and polycarbonate-polystyrene graft copolymers, terminal modified polycarbonates, polyolefins, cycloolefins and cycloolefin copolymers, and/or olefin-maleimide copolymers.

The application of a hydrophilic pattern on a hydrophobic substrate and/or the application of hydrophobic "guiding elements" on a hydrophilic substrate or any combination of it can be accomplished with flexography, lithography, gravure, solid ink coating methods, or ink-jet-printing processes.

However, the preferred fabrication method is flexography, which allows high-resolution printing on rotary presses and supports high-speed production. It is an established technology for printing on polymer film substrates and widely used in the packaging industry. The optical detection process shown in Figures 8a and 8b requires transparent and clear ink with low viscosity for the hydrophilic pattern. Low viscous inks are preferred to achieve a thin and even coating of about 2 - 4 microns. The optical window of the ink needs to be in the wavelength range suitable for the optical detection of the chemical reaction. The requirements for hydrophobic inks, apart from the hydrophobic nature, are less stringent and could be used to decorate the coagulation test element with a desired colour as well, thus non transparent inks are preferred for this step. The operation of a four-colour flexography-printing machine is established practice and provides no operational problems. The same holds for lithography device.

Most convenient for the preparation of the coagulation test element are solvent based inks, which are available in a large variety from various manufactures. Further, all such available inks could be fine tuned with additional additives and pigments to optimise the required parameters. Many of these inks are based on nitrocellulose ethanol or poly vinyl butyral ethanol mixtures and can be obtained e.g. form Sun Chemicals Inc. (35 Waterview Boulevard, Parsippany, NJ, USA) or Flint Ink Inc. (4600 Arrowhead Drive, Ann Arbor, MI, USA).

Even though solvent based or UV curing inks are applicable to prepare the coagulation test element, electron beam (EB) curing inks have some preferred properties. These inks provide highest resistance to mechanical and chemical factors, and contain 100% polymers, optionally with pigments, but no volatile organic solvents and photo initiators, which have proven to affect the stability of sensor chemistry. These positive gains in performance characteristics are derived from the ability of electrons to form cross-linked polymeric films and to penetrate the surface.

Inks used in EB curing make use of the polymerising capability of acrylic monomers and oligomers. Acrylic chemistry has a special significance in modem day inks. (6 J.T. Kunjappu. "The Emergence of Polyacrylates in Ink Chemistry," Ink World, February, 1999, p. 40.) The structure of the simplest acrylic compound, acrylic acid, is shown in the formula (I):

$$CH2=CH-COOH \qquad (I)$$

The double bond in the acrylic moiety opens up during interaction with electrons (initiation) and forms a free radical that acts on other monomers forming a chain (propagation) leading to high-molecular-weight polymers. As mentioned before, radiation induced polymerisation requires no external initiator since radiation itself generates free radicals with the result that no initiating species will be left in the coating.

A variety of acrylic monomers are available for EB curing that range from simple acrylates such as 2-phenoxyethyl acrylate and isooctyl acrylate, to pre-polymers like bisphenol A, epoxy acrylate and polyester/polyether acrylates (R. Golden. J. Coatings Technol., 69 (1997), p. 83). This curing technology allows the design of "functional inks" with the focus on the desired chemical and physical properties without the necessity of a solvent and curing chemistry required by other inks, which may complicate the design process.

Generally suitable hydrophobic inks might contain monomers, oligomers, and pre-polymers with hydrophobic

functions like isooctyl acrylates, dodecyl acrylates, styrene or silicon derivates, systems with partly fluorinated carbon chains, and additional hydrophobing additives and/or fillers such as hydrophobing agents belonging to the TEGO Phobe Series (TEGO Chemie Service, Essen Germany), hydrophobic pigments such as copper phthalocyans, carbon, graphite, or hydrophobic fillers such as silicon modified fumed silica or PTFE powders and PTFE granulates. Due to the vast variety of additives, pigments, and fillers the above suggested compounds will only have exemplary character.

Inks with hydrophilic functions can be realised from a wide selection of ethanol and water-soluble polymers and polymer mixtures thereof. Useful are polymers and polymer derivatives, copolymers and compounds base on alginate, cellulose and cellulose ester, hydroxyethyl cellulose, gum, acrylic acid, polyvinylalcohol, polyethylene-glycol, polyethyleneoxide, vinylpyrolidone, polystyrene sulfonate, poly(methyl vinyl ether/maleic acid), vinylpyrolidone/trimethylammonium copolymers, and alkyl-phosphocholine derivates. Further optimisation can be achieved with organo-modified silicone acrylates additives, which are a cross-linkable species of organo-modified polysiloxanes, and fluorinated surfactants. A general suitable coating provides a contact angle with water of typically less than 35° and a surface energy of typically more than 50 mN/m.

The second step of the production process comprises the application of the coagulation formulations, containing the coagulation stimulating reagent and additional agents to produce a printable and/or dispensable ink forming a uniform layer within the sample detection areas.

In a preferred embodiment, the amount of thromboplastin on first surface 2a of base layer 2 is precisely dosed using a suitable method such as ink jet printing. Indeed it would be obvious to those skilled in the art that other dosing techniques could be utilised for the purposes of this invention.

On all corresponding sample detection areas of the opposing surface will be furnished with the required quality control formulations containing the appropriated amount of alginate or another coagulation accelerator, EDTA or other coagulation inhibitors, and the fluorescent molecular rotor as detection aid if required for the anticipated detection regime.

Since the concentration level respectively the total amount of the coagulation stimulating reagent applied to the predetermined sample detection areas 6'a1 to 6'a3 is responsible for the sensitivity and dynamic range of the various discussed coagulation test elements, as well as the concentration level and precision of the applied quality control compounds is responsible for the accuracy of the test results, it is paramount for this application to provide coagulation test elements with a precise dosage of the above elements, compounds, and ingredients. Such precise dosage can be implemented for example using a micro dispenser system (e.g. available from Vermes Technik GmbH & Co. KG, Palnkamer Str. 18-20, D-83624 Otterfing, Germany). The coating formulations must be prepared to be highly soluble by the liquid sample medium to allow a fast and residue free reconstitution after the introduction of the sample fluid.

The next step comprises the lamination procedure, in which the base and cover layer presenting the first and second surfaces of the sample distribution system are laminated onto a centre layer, thereby defining a distance between the first and second surface of the base and cover layer. The centre layer provides a discontinuity to create a cavity for the sample distribution system in the areas where the sample distribution system is formed on the first and second surface of the base and cover layer. The patterns of high and low surface energy formed on the first and second surface of the base and cover layer must be aligned to be mostly congruent to enable the formation of a functional sample distribution system between the first and second surface.

Precise xy-registration of base and cover layers becomes a critical task for the function of the element, if this registration is not achieved, the sample distribution system will not function properly and/or will have a higher variability with regards to the specified sample volume. Registration tolerances should be within +/- 5% of the width of the hydrophilic pathways to achieve good performance.

Figure 13 shows the top view (left) and cross-section (right) of the coagulation test element and the effect of registration quality. In case of 13a the sample distribution system is assembled properly with good alignment of the hydrophilic pathways of the first 2a and second surface 4a. The result of an improperly aligned coagulation test element is given in Figure 13b. Although, the spacer between the base 2 and the cover layer 4 is identical in case of 13a and 13b the sample volume is falsely enlarged in case b, since the sample fluid covers partly the hydrophobic guiding elements of the sample distribution system. The effect is caused by the sample fluid inside the coagulation test element, which seeks to minimise the surface area exposed to air in order to gain the most favourable energetic state and therefore overriding the effect of the hydrophobic areas.

In an alternative embodiment, as shown in Figure 13c, the sample distribution system of the cover layer 4 is designed about 10% smaller as the sample distribution system of the base layer 2 thus the total sample volume of the coagulation test element is defined by the extensions of the sample distribution system of the base layer, allowing a higher tolerance for the registration process during manufacturing without compromising the precision of the required sample volume.

The application of the centre layer, which may be a double-sided adhesive tape with a preferred thickness of 80 microns or alternatively a hot melt adhesive deposited in an equivalent thickness, is less demanding because of the relatively large discontinuity in the material compared to the size of the hydrophilic pathways. Registration is especially important in continuous production lines where the substrate progresses with several meters up to tens of meters per

minute. Substrate expansion and web tension make the registration in x-direction (the direction of the web movement) more difficult than the y-direction perpendicular to the web movement.

A preparation method for flexible polymer films providing an accurate registration of the patterns of first and second surface is illustrated in Figure 14 showing parts of a continuous web production process. In a first production step according to Figure 14a, patterns of the sample distribution system 6 of the base and cover layer are printed on one web substrate 49, which represents the material of the produced coagulation test elements. As illustrated in Figure 14, the printed patterns of the sample distribution systems 6 are arranged on the web substrates 49 in such a manner that two sample distribution systems are opposite to each other left and right from a mirror line. Optionally, the sample distribution system can be linked in the areas which form the sample application areas. Thus, the position of the predetermined detection areas 6a, 6'a is fixed relative to each other and remains unaffected by the material expansion and web tension.

The dotted lines 50 indicate the future cutting lines to segregate the coagulation test elements into strips, while the dotted lines 51 indicate the mirror line of the strip artwork and the future fold line of the web substrate.

After printing the flow paths of the coagulation test element, the detection areas 6a, 6'a of the sample distribution system are coated with the required formulations. For example, the detection areas 6a of the upper row of the web substrate 49, which will represent the second surface of the coagulation test element, are coated with the quality control formulations. One of the quality control formulations (e. g. positioned in 6'a1) do not contain active compounds that either inhibit or promote the coagulation reaction and therefore deliver the determined result of the coagulation analysis, whereas the detection areas 6'a of the lower row of the web substrate 49, which will represent the first surface of the coagulation test element, are coated with the coagulation formulations containing the tissue thromboplastin to initiate the coagulation reaction. In the special cases other compounds than tissue thromboplastin will be coated on the sample detection areas 6'a, which will trigger and activate the coagulation pathway in different positions to determine the functionality of other coagulation factors.

Thereafter, an additionally layer is laminated on one of the surfaces, e. g. the surface 2a of the base layer 2, representing the centre layer 52 of the coagulation test element as shown in Figure 14b. The centre layer 52 may be formed of double-sided adhesive tape or a hot melt adhesive, which provides breakthroughs 5 exposing the sample distribution systems 6 to create cavities for the sample distribution systems in the coagulation test elements after the final assembly step.

The coagulation test element of the present invention is then assembled by folding the two sides along the mirror line 51, e. g. with help of a folding iron or other suitable equipment, as illustrated in Figure 14c creating a folded and laminated web 53 as shown in Figure 14d. Subsequently, a press roller can secure a tight connection between the centre layer, base and cover layer.

Finally, the laminated web 53 is cut or punched in to the desired product shape, whereas line 50 projects an exemplary shape of the final coagulation test strip onto the web 53 before the segregation process. With the preparation method illustrated in Figure 14 the top part of the substrate can be folded on to the bottom part without the danger of loosing the registration in the x-direction of the web and provides an easier method to get the right registration of the first and second surfaces forming the sample distribution system in comparison to single sheet process.

It will be obvious for someone skilled in the art that base and cover layer are exchangeable in the discussed embodiments without affecting the principle of the invention.

This invention provides a test system for determining the coagulation characteristics of plasma and whole blood samples consisting of a coagulation test element and a small and simple hand held meter device suitable for home and point of care settings. The coagulation test element is provided with an integrated quality control system suitable for dry reagent test strip format with a very small sample volume of about 0.5μL. The production of the inventive coagulation test element involves only a small number of uncomplicated production steps enabling an inexpensive production of the element.

## Claims

1. A test element for the determination of coagulation in a plasma or whole blood sample having a first surface (2a) and a second surface (4a) in a predetermined distance opposite from each other, said both surfaces being provided with two substantially equivalent patterns forming areas of high and low surface energy which are aligned mostly congruent, whereby the areas of high surface energy create a sample distribution system (6) with at least two coagulation detection areas (6a1, 6a2), wherein at least one of the detection areas (6a, 6'a) of the first and second surfaces (2a, 4a) is provided with at least one coagulation stimulation reagent.

2. A coagulation test element according to claim 1, wherein at least one coagulation detection area is provided with a formulation containing a coagulation accelerator which promotes a fast and complete coagulation of the sample fluid.

**3.** A coagulation test element according to claim 1, wherein at least one coagulation detection area is provided with a formulation containing a coagulation inhibitor which suppresses the coagulation in the sample fluid.

**4.** A coagulation test element according to claim 1, wherein the sample distribution system (6) comprises at least three coagulation detection areas, at least one coagulation detection area being provided with a formulation which accelerates the coagulation of the sample fluid, and at least one coagulation detection area being provided with a formulation which suppresses the coagulation in the sample fluid.

**5.** A coagulation test element according to at least one of the preceding claims, wherein the coagulation stimulating reagent(s) is/are thromboplastin and/or calcium ions.

**6.** A coagulation test element according to at least one of claims 2, 4 or 5 wherein the formulation accelerating the coagulation of the sample fluid comprises a gelling agent.

**7.** A coagulation test element according to at least one of the claims 3, 4, 5 or 6, wherein the formulation inhibiting the coagulation in the sample fluid comprises lithium heparin and/or EDTA.

**8.** A coagulation test element according to at least one of the preceding claims, wherein at least one of the first and second surfaces 2a, 4a of the detection area(s) (6a, 6'a) is/are provided with a compound allowing the determination of the coagulation reaction by transmission or absorbance photometry.

**9.** A coagulation test element according to at least one of the preceding claims, wherein at least one of the first and second surfaces 2a, 4a of the detection area(s) (6a, 6'a) is/are provided with (a) compound(s) allowing the determination of the coagulation reaction by fluorescence.

**10.** A coagulation test element according to claim 9, wherein the compound(s) allowing the determination of the coagulation reaction by fluorescence is/are (a) fluorescent molecular rotor(s).

**11.** A method for preparing a coagulation test element comprising the steps:

- generating areas of high and low surface energy on a base layer (2) having a first surface (2a), the areas of high surface energy forming a hydrophilic sample distribution system (6) with at least two predetermined detection areas (6a1, 6a2),
- generating a corresponding pattern of areas of high and low surface energy on a cover layer (4) having a second surface (4a),
- coating at least one of the predetermined detection areas (6a, 6a') of the first surface (2a) and/or second surface (4a) with at least one coagulation stimulation reagent,
- applying the layers of first and second surfaces to the opposite sites of a centre layer (3) having a discontinuity (5) which provides a cavity for the sample distribution system (6) formed by the areas of high surface energy on the first and second surfaces (2a, 4a) of the first and second layer (2, 4).

**12.** A method according to claim 11 comprising the additional steps of:

- coating at least one of the first and second surface 2a, 4a of a further coagulation detection area (6a2) with a formulation containing a coagulation accelerator which promotes a fast and complete coagulation of the sample fluid,
- coating at least one of the first and second surface 2a, 4a of an other coagulation detection area (6a3) with a formulation containing a coagulation inhibitor which suppresses the coagulation in the sample fluid.

**13.** A method according to claim 11 or 12 comprising the additional step of:

- coating at least one of the first and second surface 2a, 4a of the coagulation detection area(s) with (a) compound (s) allowing the determination of the coagulation reaction by fluorescence.

**14.** A method for preparing a coagulation test element according to one of claims 11 to 13, wherein said areas of high surface energy are created by applying a water insoluble hydrophilic composition on the first and second surfaces (2a, 4a).

**15.** A method for preparing a coagulation test element according to one of claims 11 to 14, wherein said areas of low surface energy are created by applying hydrophobic compositions on the first and second surfaces (2a, 4a).

**16.** A method for preparing a coagulation test element according to one of claims 14 and 15, wherein said hydrophilic and/or hydrophobic composition(s) is/are printed on the first and second surfaces (2a, 4a) by the means of flexography, lithography, gravure, solid ink coating methods, or ink-jet-printing.

**17.** A method for preparing a coagulation test element according to one of claims 11 to 16, wherein said coagulation stimulating reagent(s) and/or quality control formulation(s) and/or fluorescence detection aid(s) is/are coated on the detection areas (6a, 6'a) of first and/or second surfaces (2a, 4a) by micro-contact printing or micro dispensing.

**18.** A coagulation test system for the determination of coagulation in a plasma or whole blood sample comprising:

- a coagulation test element according to one of the claims 1 to 10,
- detection means for detecting changes of light absorbance or fluorescence in the serum or whole blood sample fluid located in the predetermined detection area(s),
- processing means for processing the data from the light detection means and calculating the coagulation time and/or INR value, and
- display means for indicating the output values to the user.

**19.** A method for determining the coagulation in a serum or whole blood sample fluid, said method comprising:

- applying a serum or whole blood sample fluid to a coagulation test element according to one of the claims 1 to 10,
- inserting the coagulation test element into a meter device including detection and processing means,
- reading the output values on a display means.

**Patentansprüche**

**1.** Testelement zur Bestimmung der Koagulation in einer Plasma- oder Gesamtblutprobe, wobei das Testelement eine erste Oberfläche (2a) und eine zweite Oberfläche (4a) aufweist, die einander in einem vorbestimmten Abstand gegenüber liegen, wobei die beiden Oberflächen zwei im Wesentlichen äquivalente Muster aufweisen, die Bereiche hoher und niedriger Oberflächenenergie bilden, welche höchst kongruent ausgerichtet sind, wobei die Bereiche hoher Oberflächenenergie ein Probenverteilungssystem (6) mit wenigstens zwei Koagulations-Detektionsbereichen (6a1, 6a2) bilden, wobei wenigstens einer der Detektionsbereiche (6a, 6'a) der ersten und zweiten Oberflächen (2a, 4a) mit wenigstens einem Koagulations-Stimulations-Reagenz versehen ist.

**2.** Koagulations-Testelement gemäß Anspruch 1, wobei wenigstens ein Koagulations-Detektionsbereich mit einer Formulierung versehen ist, welche einen Koagulations-Beschleuniger enthält, der eine schnelle und vollständige Koagulation der Probenflüssigkeit fördert.

**3.** Koagulations-Testelement gemäß Anspruch 1, wobei wenigstens ein Koagulations-Detektionsbereich mit einer Formulierung versehen ist, welche einen Koagulations-Inhibitor enthält, der die Koagulation in der Probenflüssigkeit unterdrückt.

**4.** Koagulations-Testelement gemäß Anspruch 1, wobei das Probenverteilungssystem (6) wenigstens drei Koagulations-Detektionsbereiche umfasst, wobei wenigstens ein Koagulations-Detektionsbereich mit einer Formulierung versehen ist, welche die Koagulation der Probenflüssigkeit fördert, und wobei wenigstens ein Koagulations-Detektionsbereich mit einer Formulierung versehen ist, welche die Koagulation in der Probenflüssigkeit unterdrückt.

**5.** Koagulations-Testelement gemäß wenigstens einem der vorhergehenden Ansprüche, wobei das/die Koagulations-Stimulations-Reagenz(ien) Thromboplastin und/oder Calcium-Ionen ist/sind.

**6.** Koagulations-Testelement gemäß wenigstens einem der Ansprüche 2, 4 oder 5, wobei die Formulierung, welche die Koagulation der Probenflüssigkeit beschleunigt, ein Geliermittel umfasst.

**7.** Koagulations-Testelement gemäß wenigstens einem der Ansprüche 3, 4, 5 oder 6, wobei die Formulierung, welche die Koagulation in der Probenflüssigkeit inhibiert, Lithium-Heparin und/oder EDTA umfasst.

**8.** Koagulations-Testelement gemäß wenigstens einem der vorhergehenden Ansprüche, wobei wenigstens eine der ersten und zweiten Oberflächen 2a, 4a des/der Detektionsbereich(s)/e (6a, 6'a) mit einer Verbindung versehen ist/sind, welche die Bestimmung der Koagulationsreaktion durch Transmissions- oder Absorbanz-Photometrie ermöglicht.

**9.** Koagulations-Testelement gemäß wenigstens einem der vorhergehenden Ansprüche, wobei wenigstens eine der ersten und zweiten Oberflächen 2a, 4a des/der Detektionsbereich(s)/e (6a, 6'a) mit (einer) Verbindung(en) versehen ist/sind, welche die Bestimmung der Koagulationsreaktion durch Fluoreszenz ermöglicht/ermöglichen.

**10.** Koagulations-Testelement gemäß Anspruch 9, wobei die Verbindung(en), welche die Bestimmung der Koagulationsreaktion durch Fluoreszenz ermöglicht/ermöglichen, (ein) fluoreszierende(r) molekulare(r) Rotor(en) ist/sind.

**11.** Verfahren zur Herstellung eines Koagulations-Testelements, umfassend die Schritte:

- Erzeugen von Bereichen hoher und niedriger Oberflächenenergie auf einer Basisschicht (2), welche eine erste Oberfläche (2a) aufweist, wobei die Bereiche hoher Oberflächenenergie ein hydrophiles Probenverteilungssystem (6) mit wenigstens zwei vorbestimmten Detektionsbereichen (6a1, 6a2) bilden,
- Erzeugen eines entsprechenden Musters von Bereichen hoher und niedriger Oberflächenenergie auf einer Deckschicht (4), welche eine zweite Oberfläche (4a) aufweist,
- Beschichten wenigstens eines der vorbestimmten Detektionsbereiche (6a, 6a') der ersten Oberfläche (2a) und/oder der zweiten Oberfläche (4a) mit wenigstens einem Koagulations-Stimulations-Reagenz,
- Aufbringen der Schichten der ersten und zweiten Oberflächen auf die entgegen gesetzten Seiten einer Mittelschicht (3), welche eine Diskontinuität (5) aufweist, die einen Hohlraum für das Probenverteilungssystem (6) bereitstellt, welches durch die Bereiche hoher Oberflächenenergie auf den ersten und zweiten Oberflächen (2a, 4a) der ersten und zweiten Schicht (2, 4) gebildet wird.

**12.** Verfahren gemäß Anspruch 11, umfassend die zusätzlichen Schritte:

- Beschichten wenigstens einer der ersten und zweiten Oberflächen 2a, 4a eines weiteren Koagulations-Detektionsbereichs (6a2) mit einer Formulierung, welche einen Koagulations-Beschleuniger enthält, der eine schnelle und vollständige Koagulation der Probenflüssigkeit fördert,
- Beschichten wenigstens einer der ersten und zweiten Oberflächen 2a, 4a eines anderen Koagulations-Detektionsbereichs (6a3) mit einer Formulierung, welche einen Koagulations-Inhibitor enthält, der die Koagulation in der Probenflüssigkeit unterdrückt.

**13.** Verfahren gemäß Anspruch 11 oder 12, umfassend den zusätzlichen Schritt:

- Beschichten wenigstens einer der ersten und zweiten Oberflächen 2a, 4a des/der Koagulations-Detektionsbereich(s)/e mit (einer) Formulierung(en), welche die Bestimmung der Koagulationsreaktion durch Fluoreszenz ermöglicht/ermöglichen.

**14.** Verfahren zur Herstellung eines Koagulations-Testelements gemäß einem der Ansprüche 11 bis 13, wobei die Bereiche hoher Oberflächenenergie durch Auftragen einer in Wasser unlöslichen hydrophilen Zusammensetzung auf die ersten und zweiten Oberflächen (2a, 4a) erzeugt werden.

**15.** Verfahren zur Herstellung eines Koagulations-Testelements gemäß einem der Ansprüche 11 bis 14, wobei die Bereiche niedriger Oberflächenenergie durch Auftragen hydrophober Zusammensetzungen auf die ersten und zweiten Oberflächen (2a, 4a) erzeugt werden.

**16.** Verfahren zur Herstellung eines Koagulations-Testelements gemäß einem der Ansprüche 14 und 15, wobei die hydrophile(n) und/oder hydrophobe(n) Zusammensetzung(en) auf die ersten und zweiten Oberflächen (2a, 4a) mittels Flexographie-, Lithographie-, Tiefdruck-, Festtinten-Druckverfahren oder Tintenstrahldruck gedruckt werden.

**17.** Verfahren zur Herstellung eines Koagulations-Testelements gemäß einem der Ansprüche 11 bis 16, wobei das/die Koagulations-Stimulations-Reagenz(ien) und/oder die Formulierung(en) zur Qualitätskontrolle und/oder die Fluoreszenz-Detektionshilfe(n) auf die Detektionsbereiche (6a, 6'a) der ersten und zweiten Oberflächen (2a, 4a) durch Mikrokontaktdruck oder "Mikrodispensing" aufgetragen wird/werden.

**18.** Koagulations-Testsystem zur Bestimmung der Koagulation in einer Plasma- oder Gesamtblutprobe, umfassend:

- ein Koagulations-Testelement gemäß einem der Ansprüche 1 bis 10,
- Detektionsmittel zur Detektion von Änderungen der Lichtabsorbanz oder Fluoreszenz in der Serum- oder Gesamtblutprobenflüssigkeit, die sich in dem/den vorbestimmten Detektionsbereich(en) befindet,
- Verarbeitungsmittel zur Verarbeitung der Daten von dem Lichtdetektionsmittel und Berechnen der Koagulationszeit und/oder des INR-Werts, und
- Anzeigemittel zum Anzeigen der Ausgabe-Werte an den Anwender.

**19.** Verfahren zur Bestimmung der Koagulation in einer Serum- oder Gesamtblutprobenflüssigkeit, wobei das Verfahren umfasst:

- Auftragen einer Serum- oder Gesamtblutprobenflüssigkeit auf ein Koagulations-Testelement gemäß einem der Ansprüche 1 bis 10,
- Einsetzen des Koagulations-Testelements in ein Messgerät, welches Detektions- und Verarbeitungsmittel umfasst,
- Ablesen der Ausgabe-Werte auf einem Anzeigemittel.

## Revendications

**1.** Elément de test pour la détermination de la coagulation dans un échantillon de plasma ou de sang total ayant une première surface (2a) et une deuxième surface (4a) opposée l'une à l'autre par une distance prédéterminée, lesdites deux surfaces étant dotées de deux motifs sensiblement équivalents formant des aires d'énergie de surface élevée et faible qui sont alignées et quasiment en correspondance, les aires d'énergie de surface élevée créant ainsi un système de distribution d'échantillon (6) avec au moins deux zones de détection de la coagulation (6a1, 6a2), au moins une des zones de détection (6a, 6'a) des première et deuxième surfaces (2a, 4a) étant dotée d'au moins un réactif de stimulation de la coagulation.

**2.** Elément de test de coagulation selon la revendication 1, dans lequel au moins une zone de détection de la coagulation est dotée d'une formulation contenant un accélérateur de coagulation qui promeut une coagulation complète et rapide de l'échantillon de fluide.

**3.** Elément de test de coagulation selon la revendication 1, dans lequel au moins une zone de détection de la coagulation est dotée d'une formulation contenant un inhibiteur de coagulation qui empêche la coagulation dans l'échantillon de fluide.

**4.** Elément de test de coagulation selon la revendication 1, dans lequel le système de distribution d'échantillon (6) comprend au moins trois zones de détection de la coagulation, au moins une zone de détection de la coagulation étant dotée d'une formulation qui accélère la coagulation de l'échantillon de fluide, et au moins une zone de détection de la coagulation étant dotée d'une formulation qui empêche la coagulation dans l'échantillon de fluide.

**5.** Elément de test de coagulation selon au moins l'une des revendications précédentes, dans lequel le(s) réactif(s) de stimulation de la coagulation est/sont la thromboplastine et/ou les ions calcium.

**6.** Elément de test de coagulation selon au moins l'une des revendications 2, 4 ou 5, dans lequel la formulation accélérant la coagulation de l'échantillon de fluide comprend un agent gélifiant.

**7.** Elément de test de coagulation selon au moins l'une des revendications 3, 4, 5 ou 6, dans lequel la formulation inhibant la coagulation dans l'échantillon de fluide comprend de l'héparine lithium et/ou de l'EDTA.

**8.** Elément de test de coagulation selon au moins l'une des revendications précédentes, dans lequel au moins une des première et deuxième surfaces (2a, 4a) de la (des) zone(s) de détection (6a, 6'a) est dotée d'un composé permettant la détermination de la réaction de coagulation par photométrie de transmission ou d'absorption.

**9.** Elément de test de coagulation selon au moins l'une des revendications précédentes, dans lequel au moins une des première et deuxième surfaces (2a, 4a) de la (des) zone(s) de détection (6a, 6'a) est dotée d'un (de) composé (s) permettant la détermination de la réaction de coagulation par fluorescence.

**10.** Elément de test de coagulation selon la revendication 9, dans lequel le(s) composé(s) permettant la détermination de la réaction de coagulation par fluorescence est un (des) rotor(s) moléculaire(s) fluorescent(s).

**11.** Procédé de préparation d'un élément de test de coagulation qui comprend les étapes consistant à :

- générer des aires d'énergie de surface élevée et faible sur une couche de base (2) ayant une première surface (2a), les aires d'énergie de surface élevée formant un système de distribution d'échantillon hydrophile (6) avec au moins deux zones de détection prédéterminées (6a1, 6a2),
- générer un motif correspondant d'aires d'énergie de surface élevée et faible sur une couche de couverture (4) ayant une deuxième surface (4a),
- revêtir au moins l'une des zones de détection prédéterminées (6a, 6a') de la première surface (2a) et/ou de la deuxième surface (4a) d'au moins un réactif de stimulation de la coagulation,
- appliquer les couches des première et deuxième surfaces aux côtés opposés d'une couche centrale (3) ayant une discontinuité (5) qui fournit une cavité pour le système de distribution d'échantillon (6) formé par les aires d'énergie de surface élevée sur les première et deuxième surfaces (2a, 4a) des première et deuxième couches (2, 4).

**12.** Procédé selon la revendication 11, qui comprend les étapes supplémentaires consistant à :

- revêtir au moins une des première et deuxième surfaces (2a, 4a) d'une autre zone de détection de la coagulation (6a2) d'une formulation contenant un accélérateur de coagulation qui promeut une coagulation complète et rapide de l'échantillon de fluide,
- revêtir au moins une des première et deuxième surfaces (2a, 4a) d'une autre zone de détection de la coagulation (6a3) d'une formulation contenant un inhibiteur de coagulation qui empêche la coagulation de l'échantillon de fluide.

**13.** Procédé selon la revendication 11 ou 12, qui comprend l'étape supplémentaire consistant à :

- revêtir au moins une des première et deuxième surfaces (2a, 4a) de la (des) zone(s) de détection de la coagulation d'un (de) composé(s) permettant la détermination de la réaction de coagulation par fluorescence.

**14.** Procédé de préparation d'un élément de test de coagulation selon l'une des revendications 11 à 13, dans lequel lesdites zones d'énergie de surface élevée sont créées par application d'une composition hydrophile insoluble dans l'eau sur les première et deuxième surfaces (2a, 4a).

**15.** Procédé de préparation d'un élément de test de coagulation selon l'une des revendications 11 à 14, dans lequel lesdites zones d'énergie de surface faible sont créées par application de compositions hydrophobes sur les première et deuxième surfaces (2a, 4a).

**16.** Procédé de préparation d'un élément de test de coagulation selon l'une des revendications 14 et 15, dans lequel ladite (lesdites) composition(s) hydrophile et/ou hydrophobe est/sont imprimée(s) sur les première et deuxième surfaces (2a, 4a) par les moyens de procédés de flexographie, de lithographie, de gravure, de revêtement d'encre solide, ou par impression par jet d'encre.

**17.** Procédé de préparation d'un élément de test de coagulation selon l'une des revendications 11 à 16, dans lequel ledit (lesdits) réactif(s) de stimulation de la coagulation et/ou ladite (lesdites) formulation(s) de contrôle de qualité et/ou ledit (lesdits) adjuvant(s) de détection de fluorescence sont revêtus sur les zones de détection (6a, 6'a) des première et/ou deuxième surfaces (2a, 4a) par impression par microcontact ou par micro-distribution.

**18.** Système de test de coagulation destiné à déterminer la coagulation dans un échantillon de plasma ou de sang total, qui comprend :

- un élément de test de coagulation selon l'une des revendications 1 à 10,
- des moyens de détection destinés à détecter les modifications de l'absorbance de la lumière ou de la fluorescence dans l'échantillon liquide de sérum ou de sang total situé dans la (les) zone(s) de détection prédéterminées,
- des moyens de traitement destinés à traiter les données des moyens de détection de lumière et à calculer la durée de coagulation et/ou la valeur du RIN, et
- des moyens d'affichage destinés à indiquer les valeurs calculées à l'utilisateur.

**19.** Procédé de détermination de la coagulation dans un échantillon liquide de sérum ou de sang total, ledit procédé comprenant :

- l'application d'un échantillon liquide de sérum ou de sang total à un élément de test de coagulation selon l'une des revendications 1 à 10,
- l'insertion de l'élément de test de coagulation dans un dispositif de mesure comprenant des moyens de détection et de traitement,
- la lecture des valeurs calculées sur des moyens d'affichage.

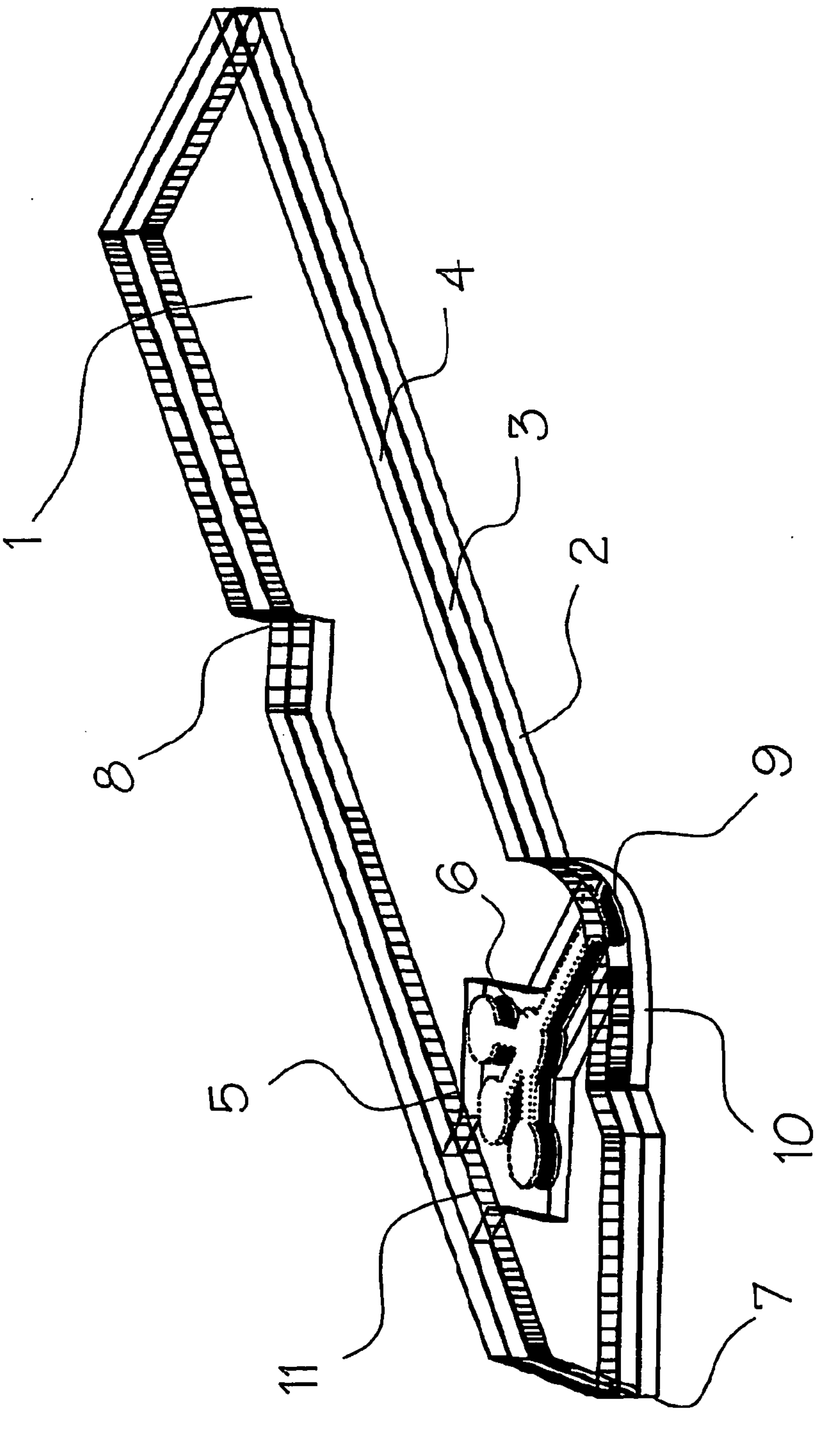


Fig. 1

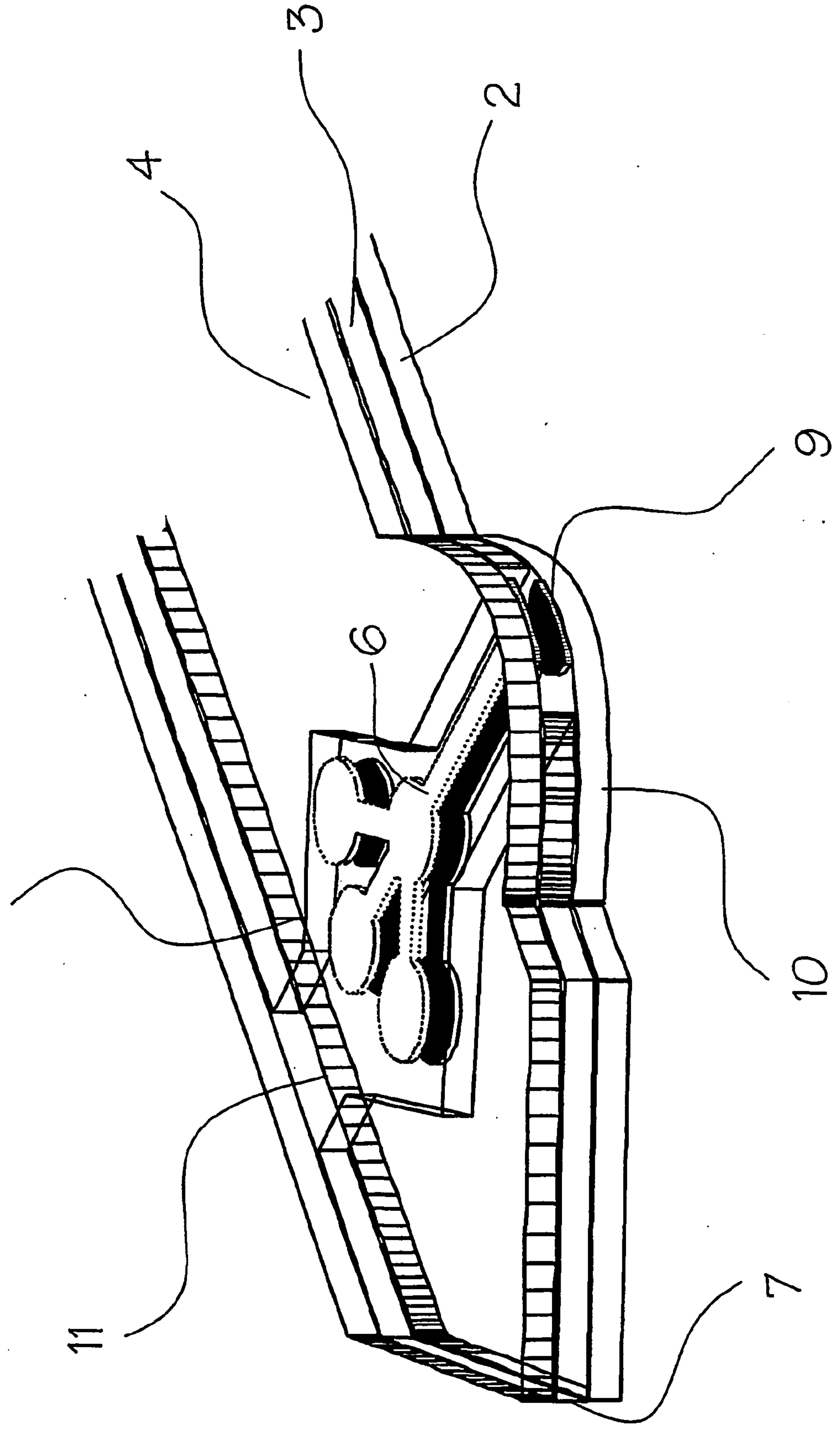


Fig. 2

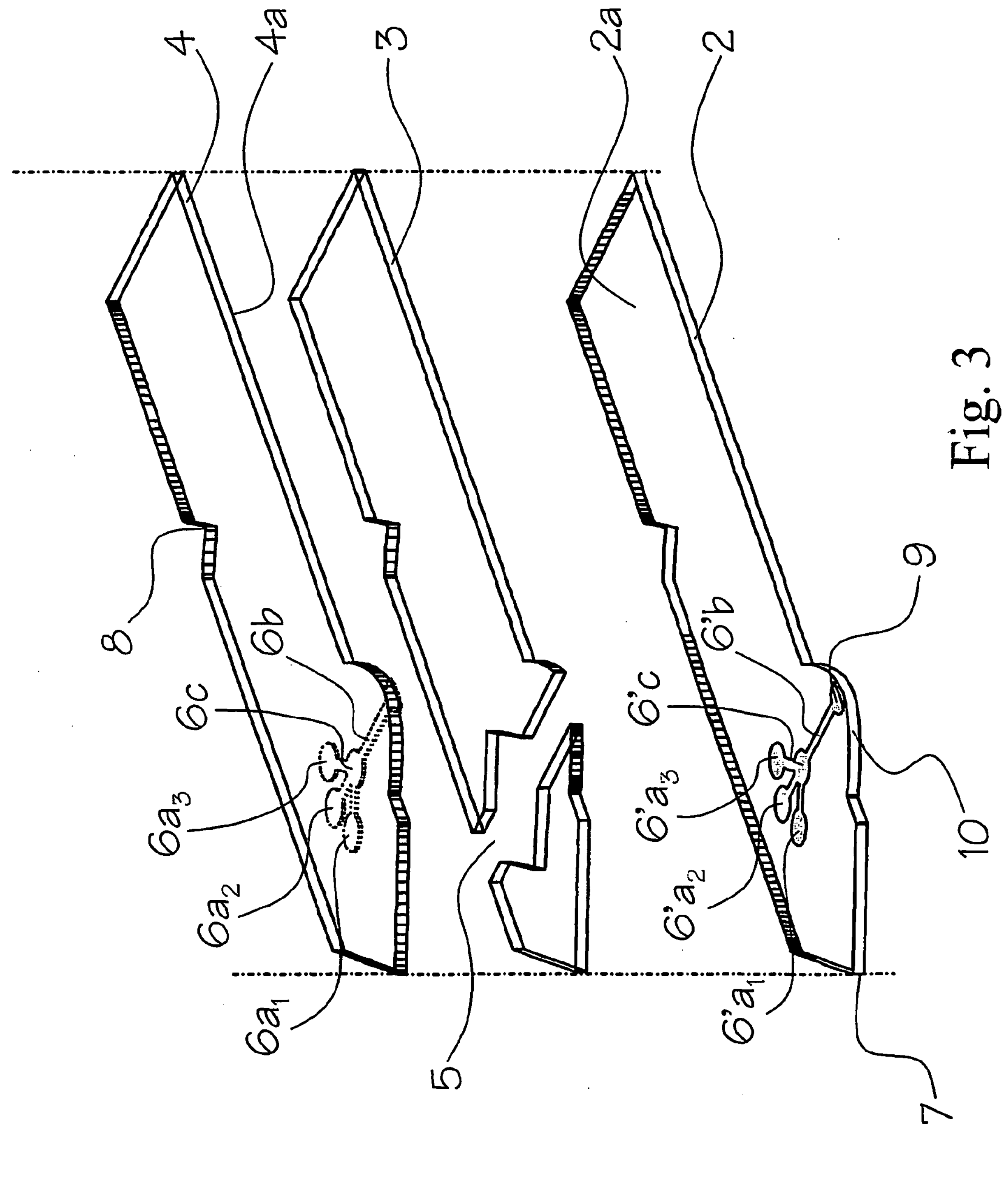


Fig. 3

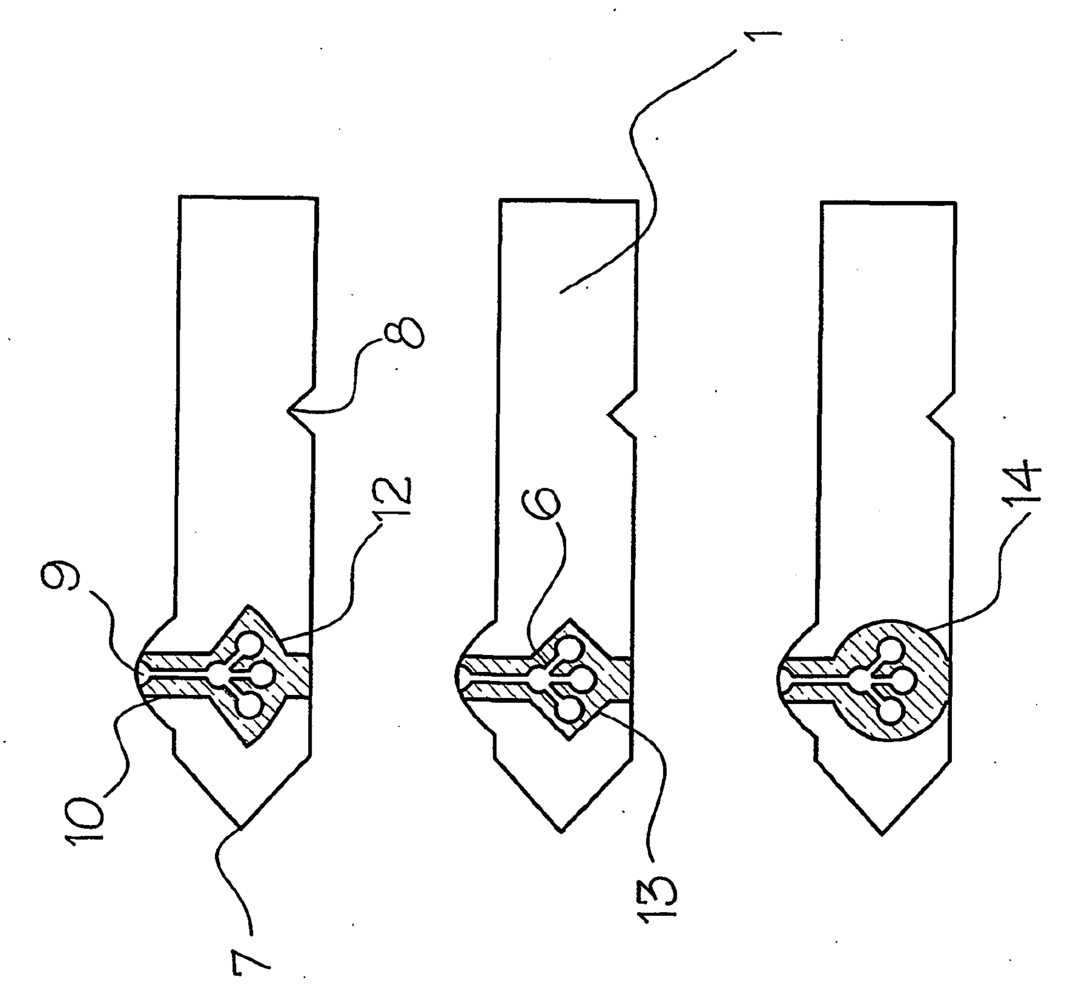


a) b) c)

Fig. 4

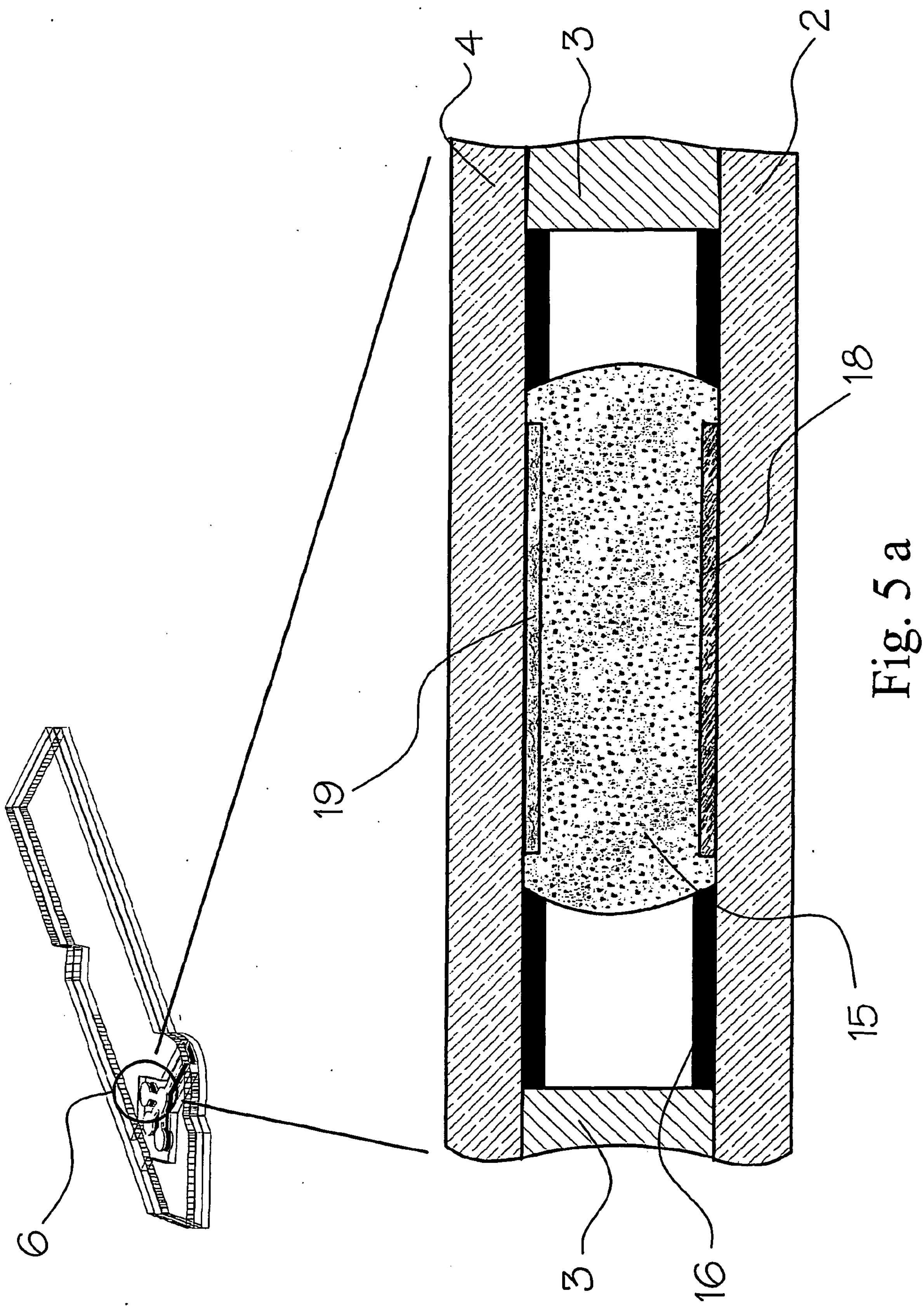


Fig. 5 a

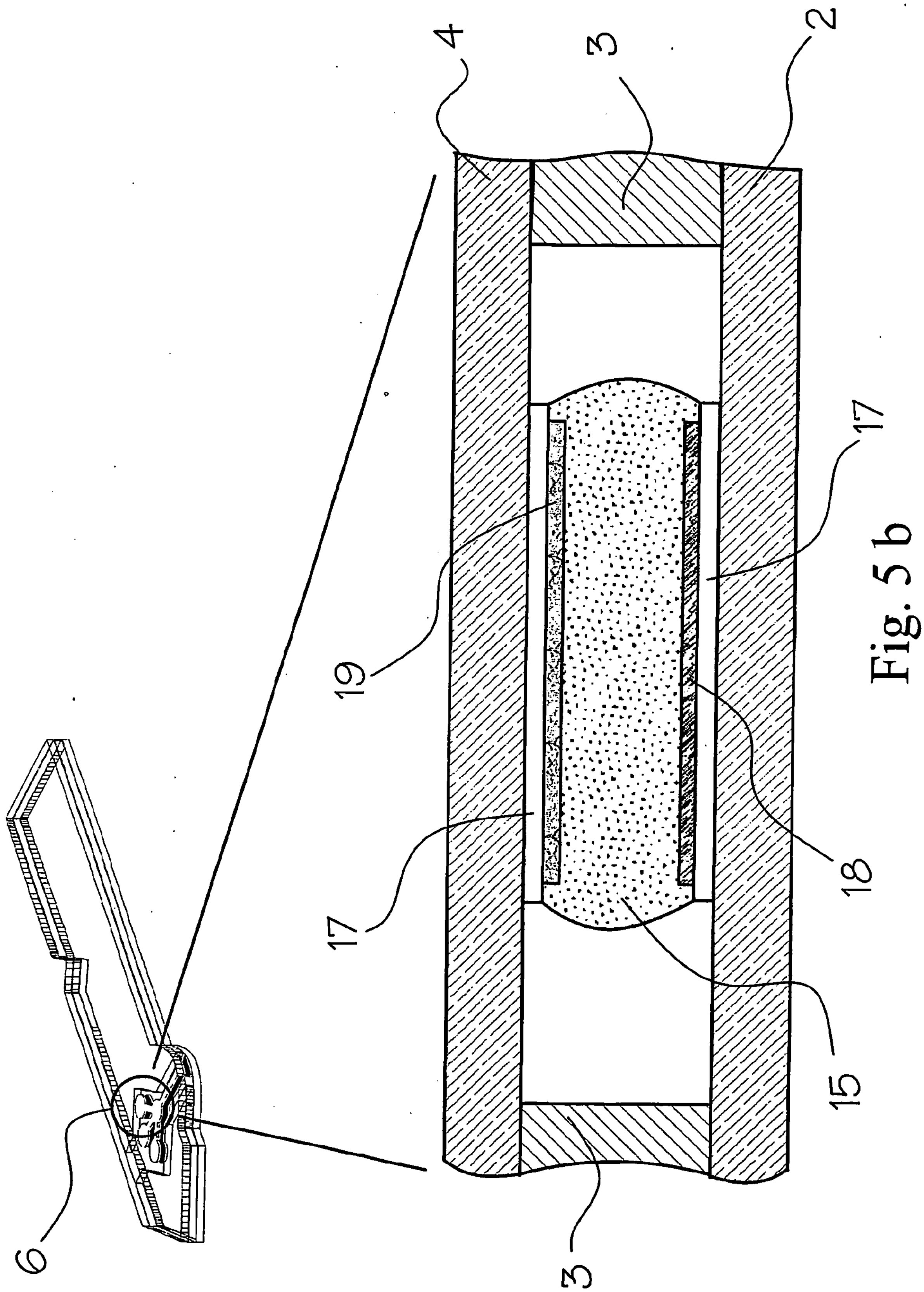


Fig. 5 b

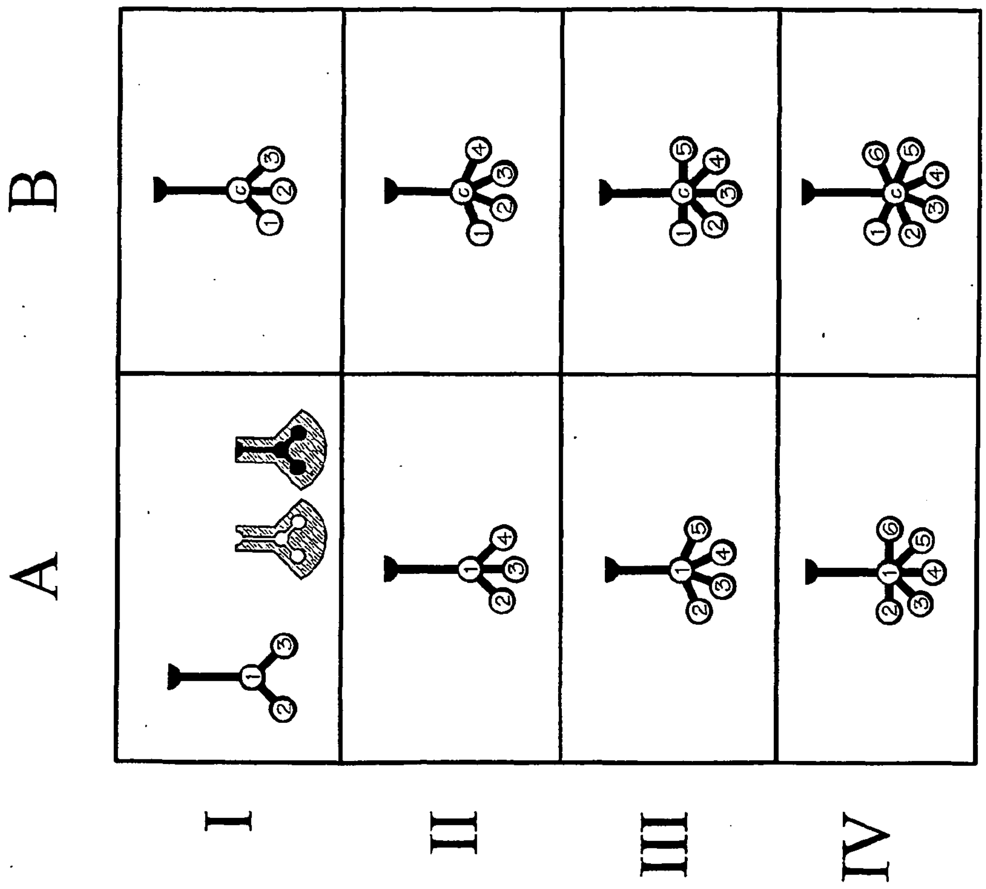


Fig. 6

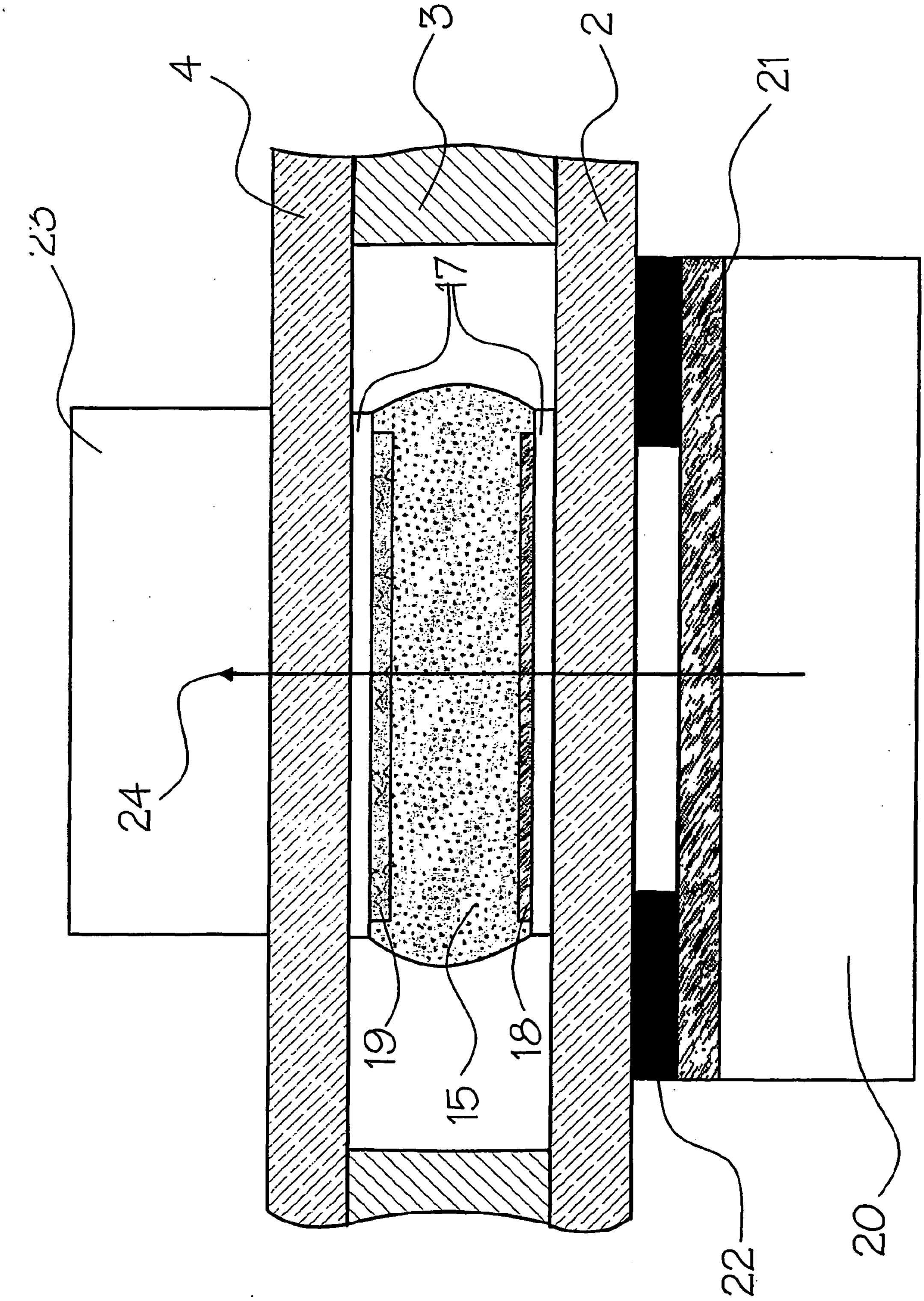


Fig. 7a

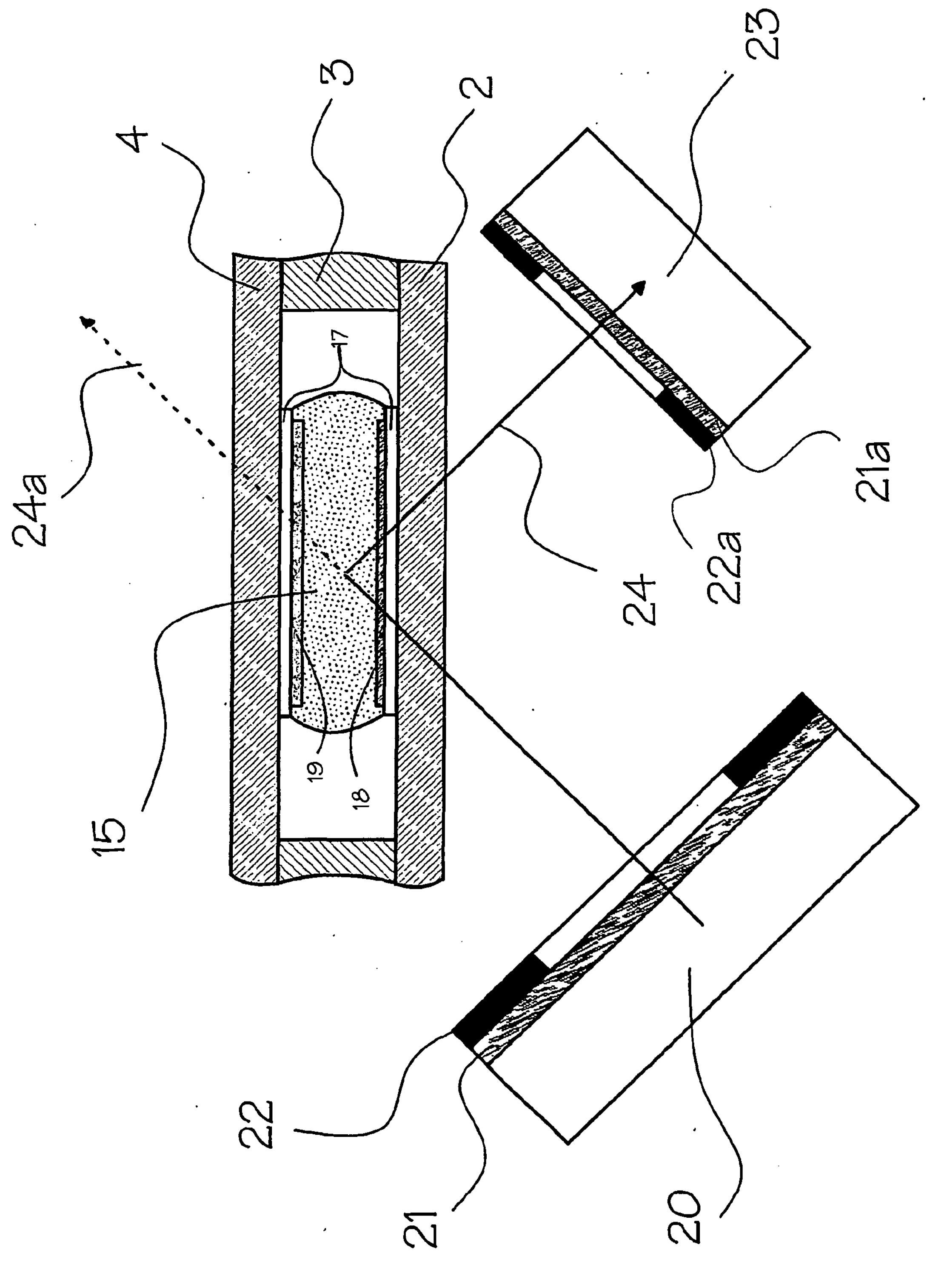


Fig. 7b

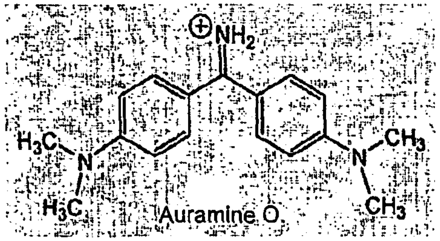

Auramine O

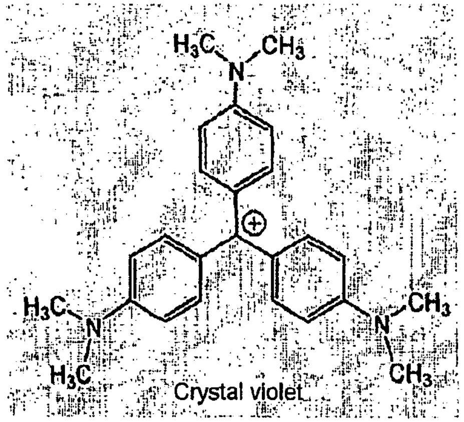

Crystal violet

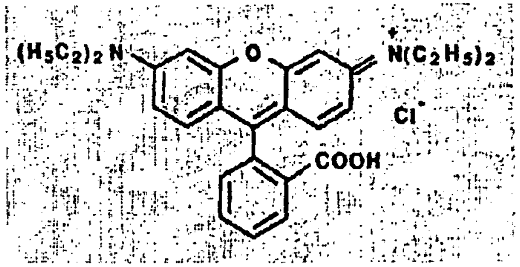

Rhodamine B

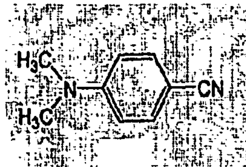

p-N,N-dimethylamino-benzonitrile (DMABN)

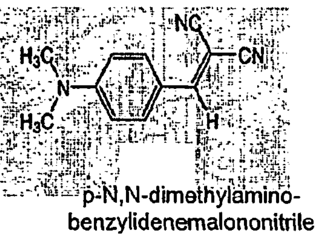

p-N,N-dimethylamino-benzylidenemalononitrile

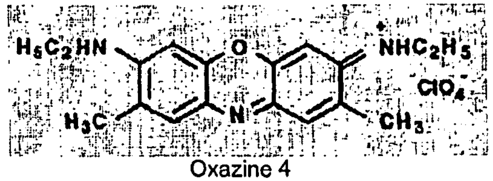

Oxazine 4

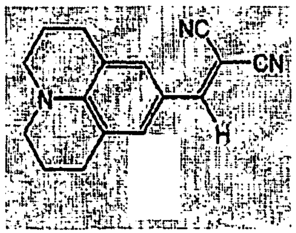

Julolidinebenzylidene-malononitrile

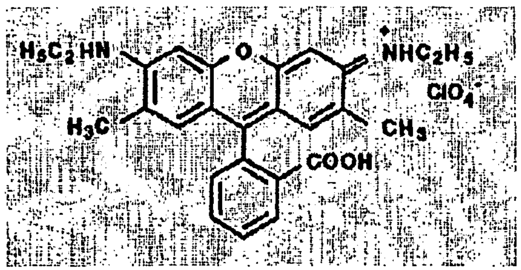

Rhodamine 19

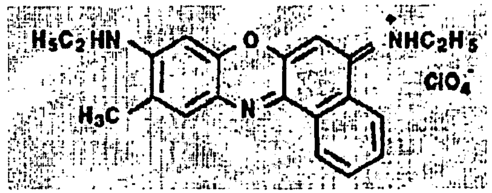

Oxazine 170

Fig. 8

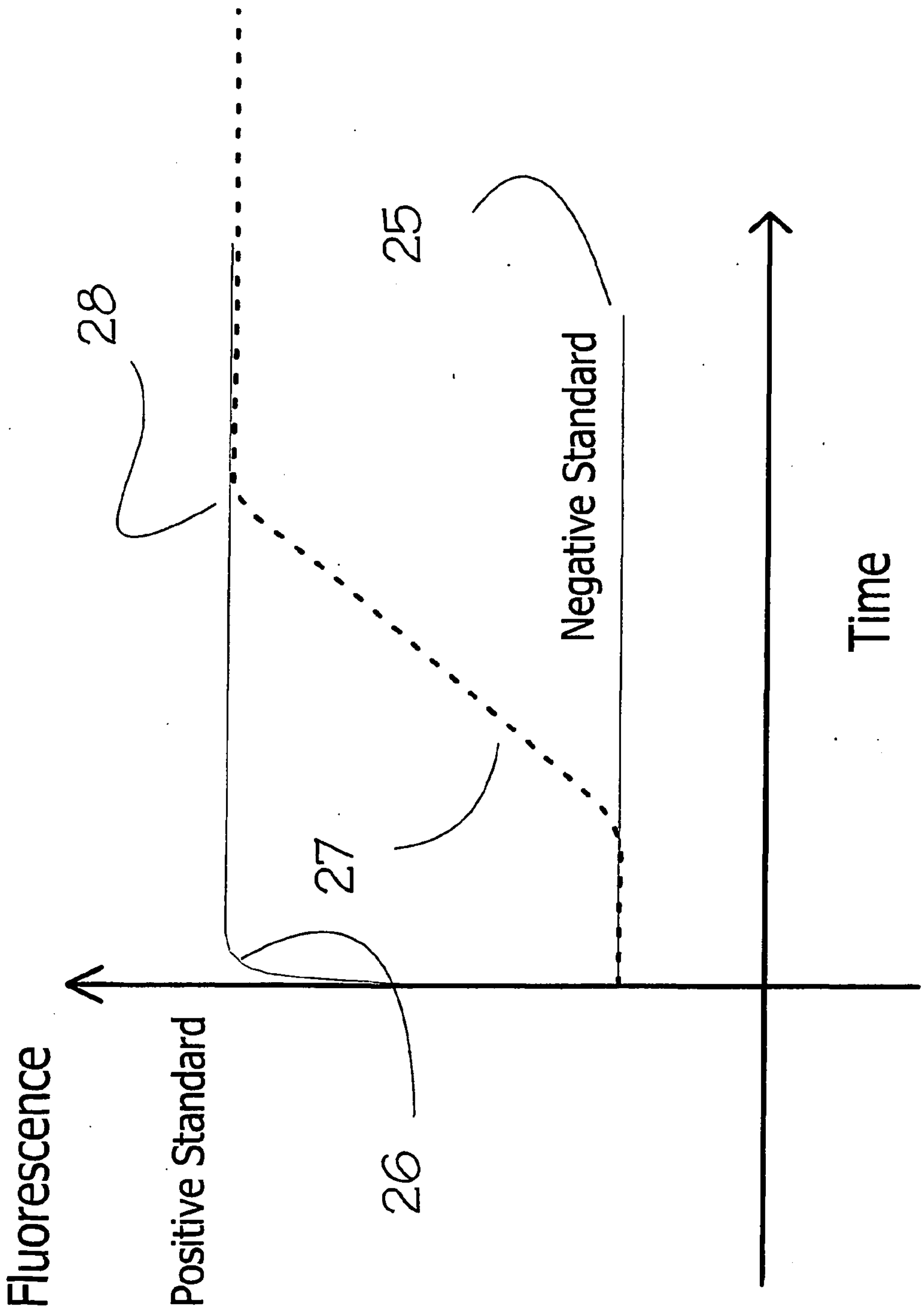
Fluorescence
Positive Standard
26
27
28
25
Negative Standard
Time

Fig. 9

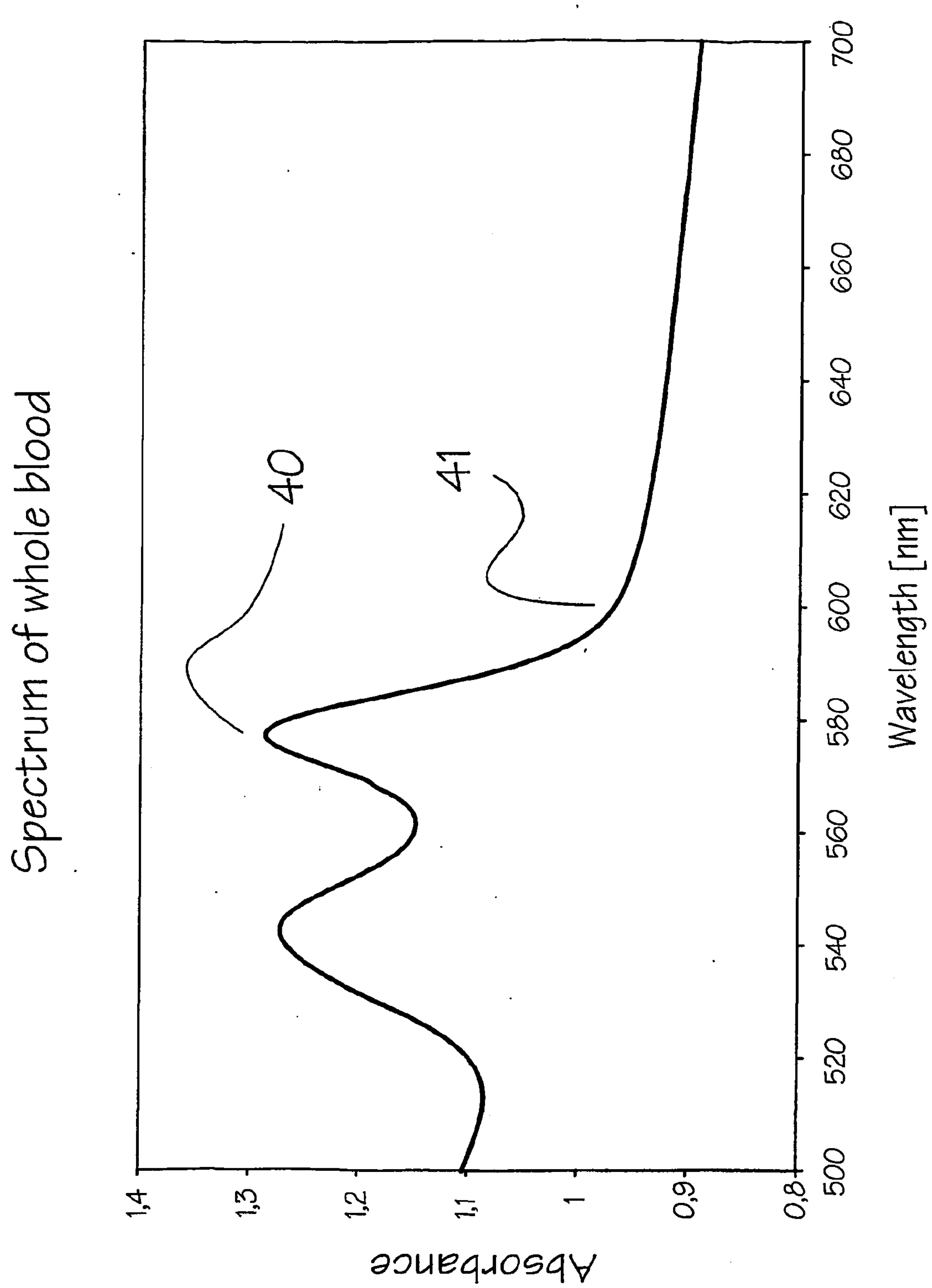
Spectrum of whole blood
40
41
Absorbance
1,4
1,3
1,2
1,1
1
0,9
0,8
500
520
540
560
580
600
620
640
660
680
700
Wavelength [nm]

Fig. 10

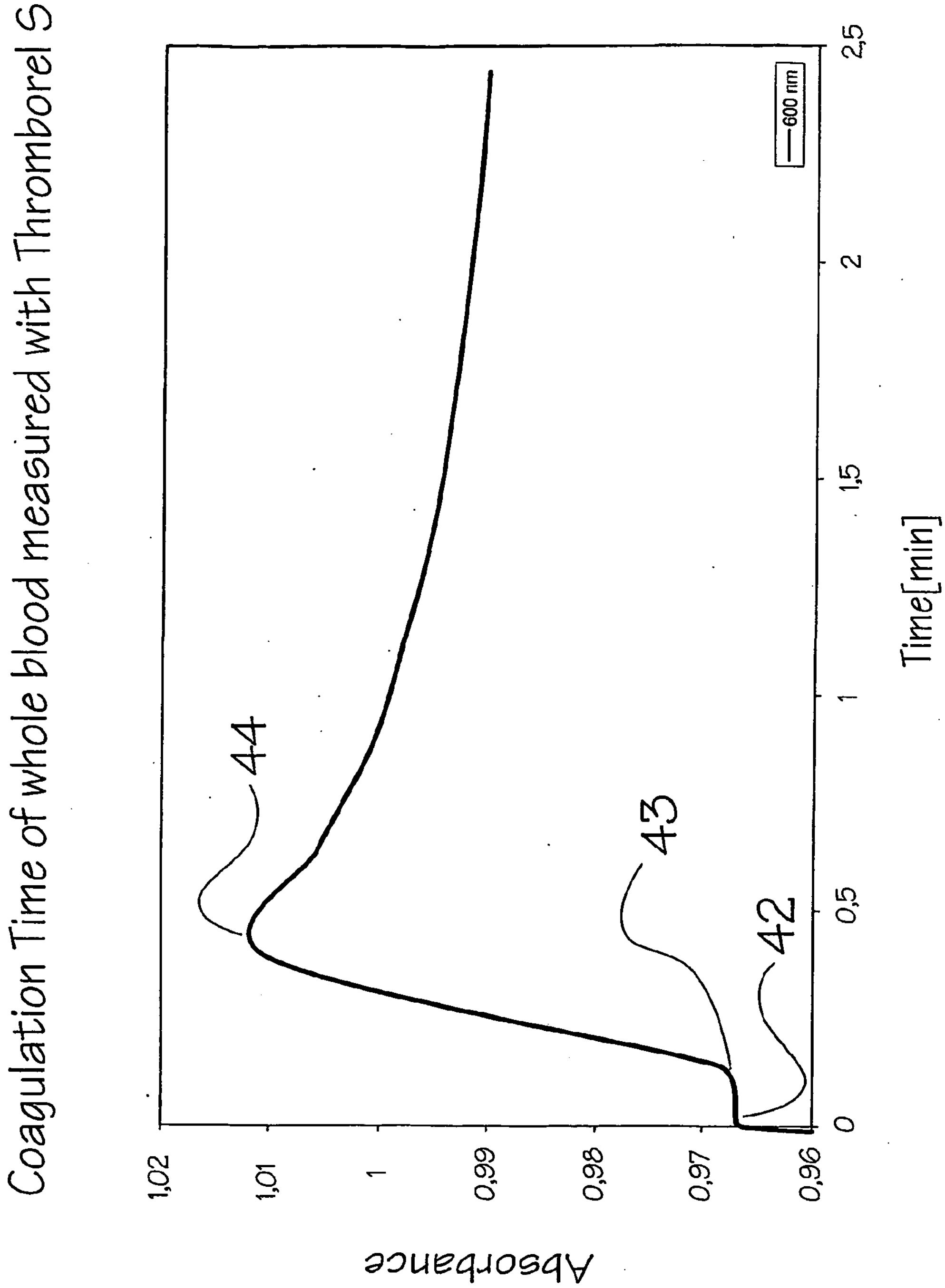
Coagulation Time of whole blood measured with Thromborel S
Absorbance
1,02
1,01
1
0,99
0,98
0,97
0,96
0
0,5
1
1,5
2
2,5
Time[min]
600 nm
42
43
44

Fig. 11

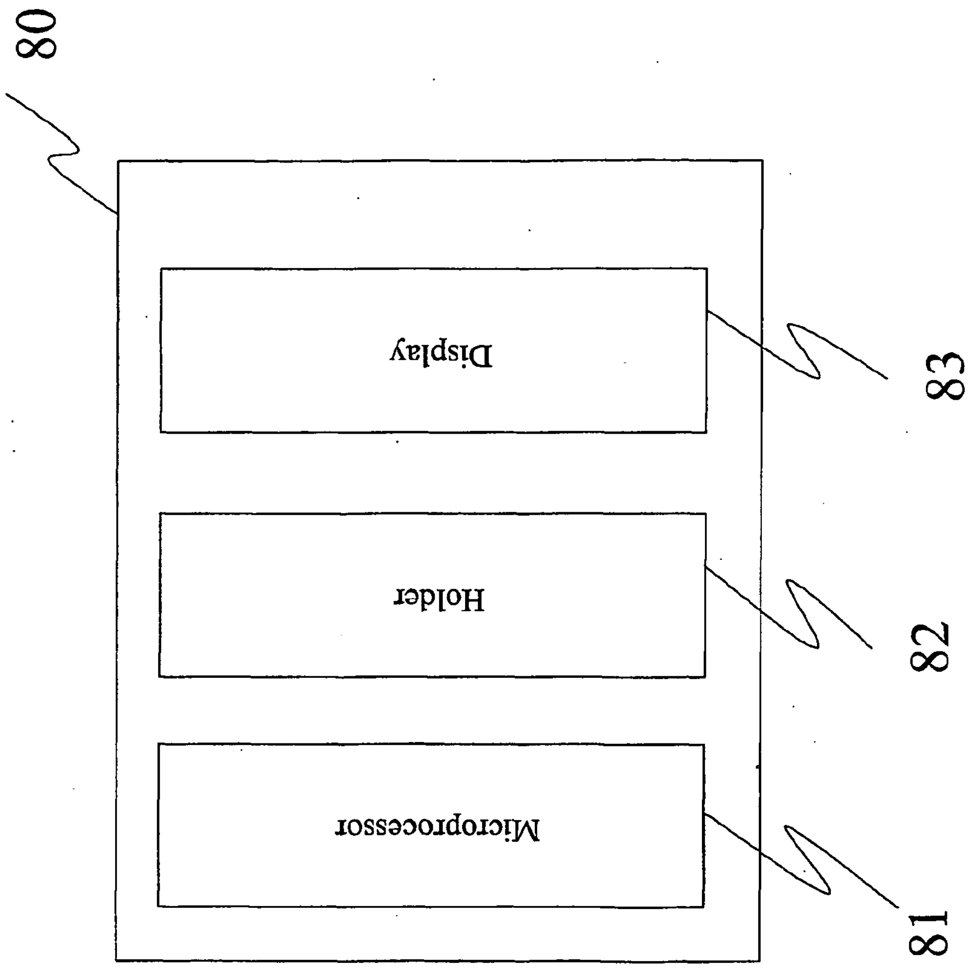


Fig. 12

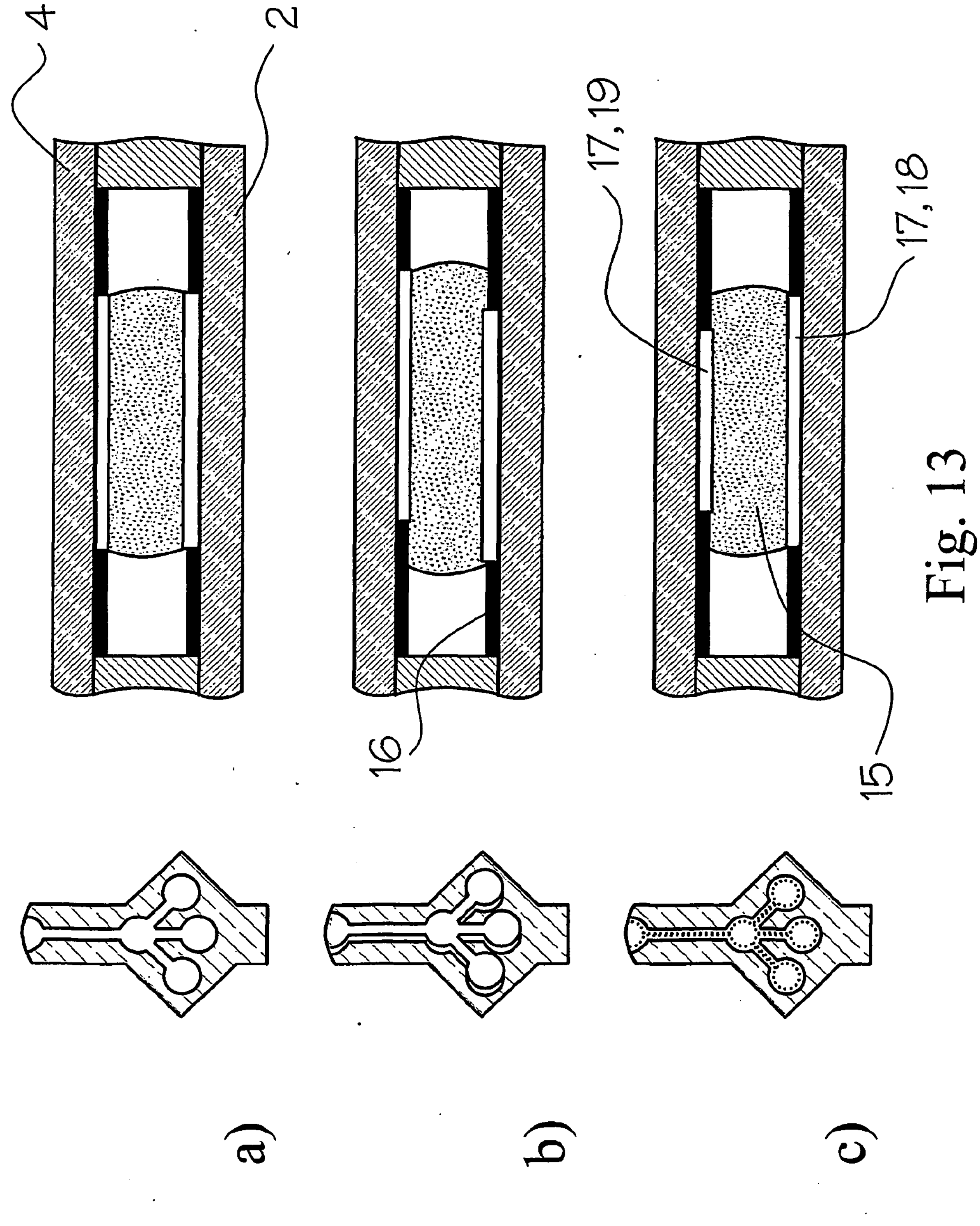


Fig. 13

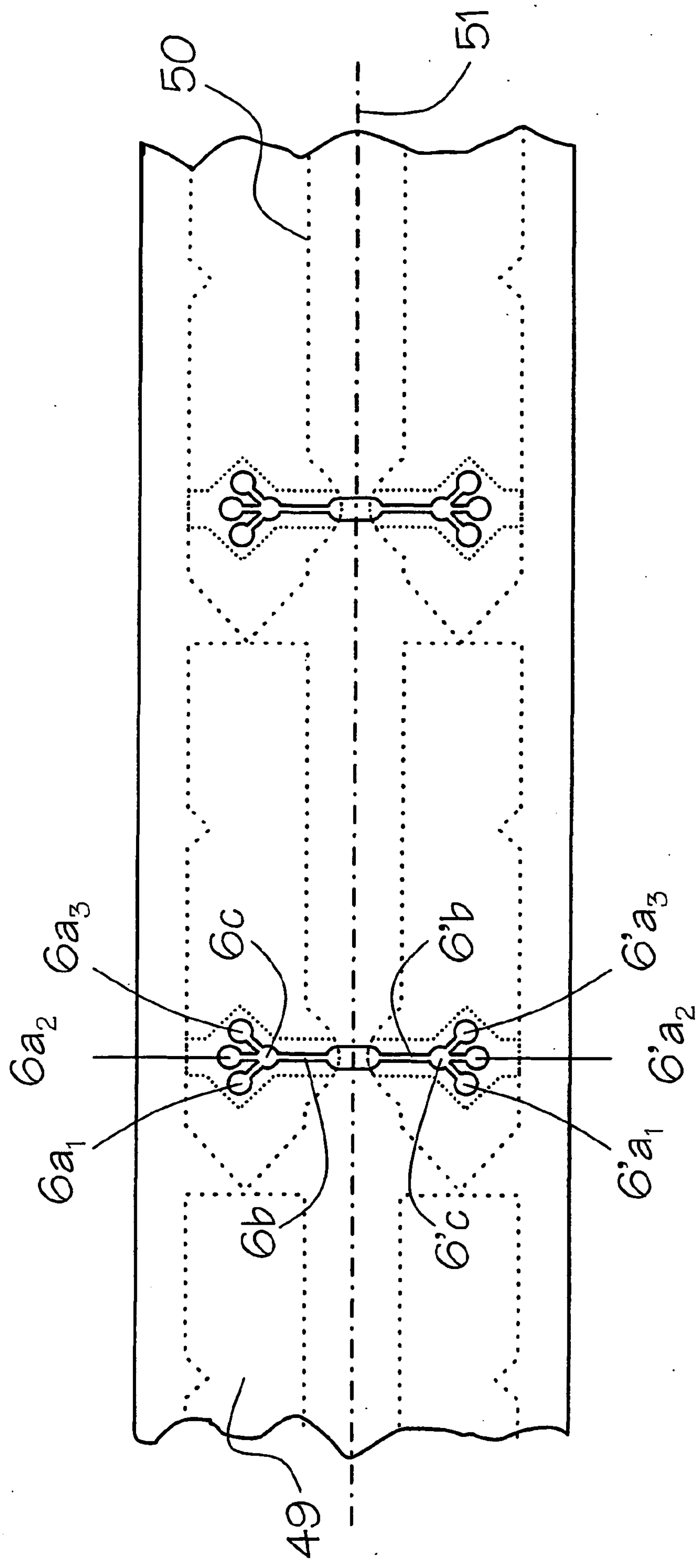


Fig. 14a

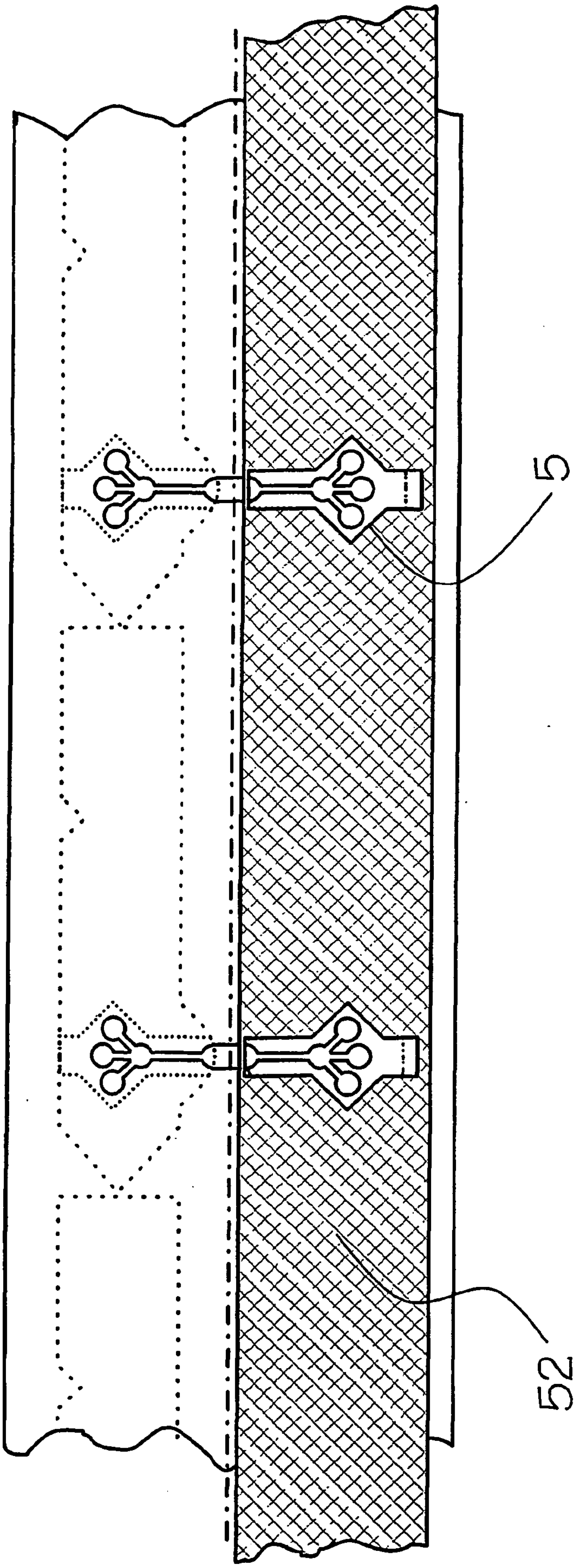


Fig. 14 b

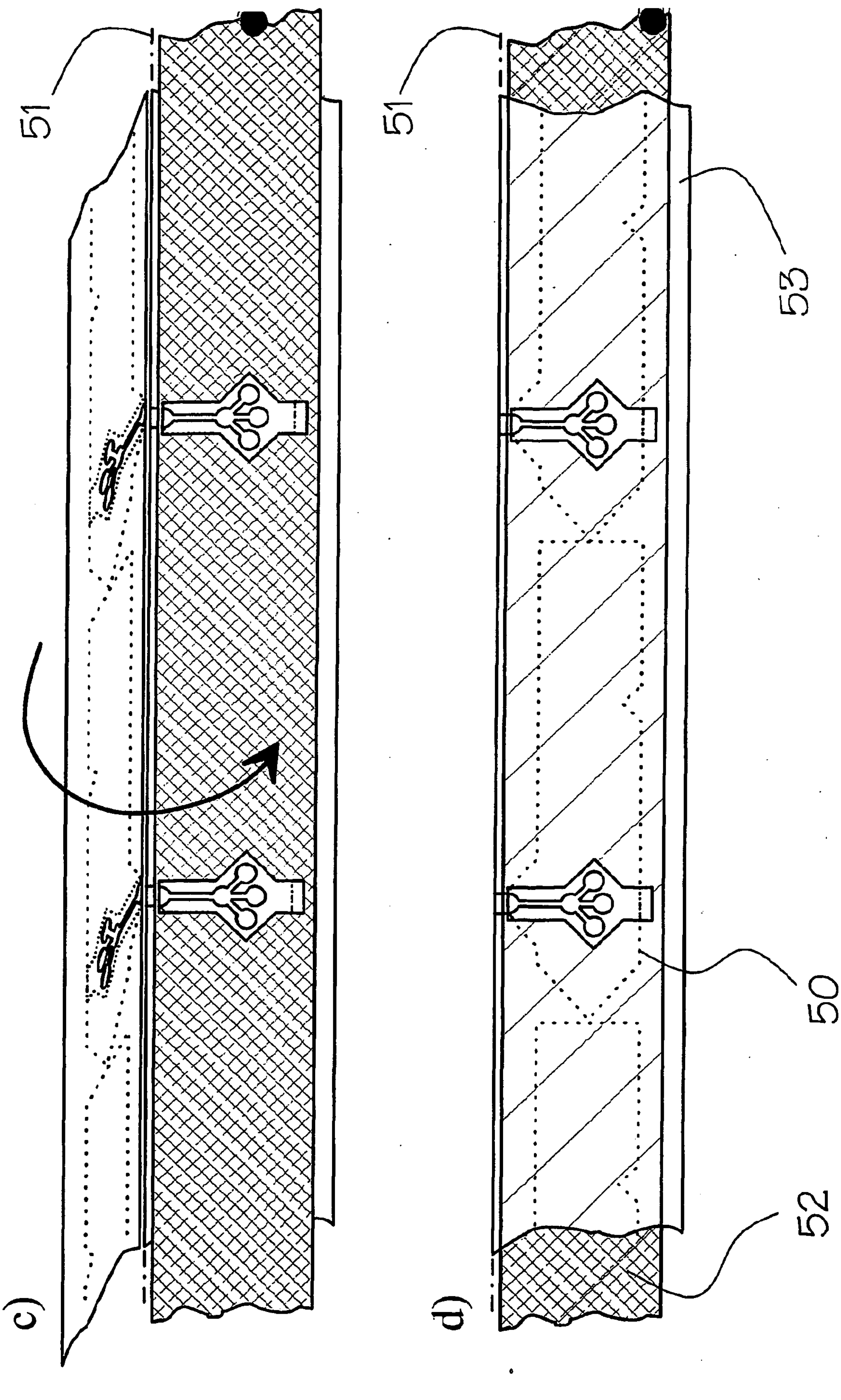


Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4252536 A
- EP 1162457 A
- US 6066504 A
- EP 974840 A
- WO 20047044560 A
- US 6084660 A
- WO 2002086472 A
- US 20020110486 A1
- US 20030031594 A1
- EP 2004002284 W
- US 2003210287 A
- WO 0056808 A
- WO 02076878 A
- US 2004031688 A


### Non-patent literature cited in the description


- The Emergence of Polyacrylates in Ink Chemistry. **J.T. Kunjappu.** Ink World. February 1999, 40
- **R. Golden.** *J. Coatings Technol.,* 1997, vol. 69, 83